(19) Europäisches Patentamt / European Patent Office / Office européen des brevets

(11) **EP 4 140 537 A1**

(12) **EUROPÄISCHE PATENTANMELDUNG**

(43) Veröffentlichungstag:
01.03.2023  Patentblatt 2023/09

(21) Anmeldenummer: 21193188.6

(22) Anmeldetag: **26.08.2021**

(51) Internationale Patentklassifikation (IPC):
**A61N 5/06** (2006.01)　　　**A61F 7/00** (2006.01)
**A47C 7/74** (2006.01)

(52) Gemeinsame Patentklassifikation (CPC):
**A61N 5/0625; A61F 7/007;** A61F 2007/0024;
A61F 2007/0088; A61N 2005/0642;
A61N 2005/0659

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Inframedic AG**
**7203 Trimmis (CH)**

(72) Erfinder:
• **PECHER, Otto**
**85653 Aying b. München (DE)**
• **ZEIGER, Thomas**
**6134 Vomp (AT)**
• **MATTHEISS, Gerd**
**7203 Trimmis (CH)**

(74) Vertreter: **Fuchs Patentanwälte Partnerschaft mbB**
**Westhafenplatz 1**
**60327 Frankfurt am Main (DE)**

(54) **VORRICHTUNG UND INFRAROTHEIZMODUL ZUM ZUFÜHREN VON WÄRME**

(57)　Die vorliegende Erfindung betrifft eine Vorrichtung zum Zuführen von Wärme an eine Hautpartie eines Lebewesens. Die vorliegende Erfindung betrifft außerdem ein Infrarotheizmodul zum Zuführen von Wärme an eine Hautpartie eines Lebewesens. Die vorliegende Erfindung betrifft ferner eine erfindungsgemäße Vorrichtung, die ein erfindungsgemäßes Infrarotheizmodul aufweist.

Fig. 1a

**Beschreibung**

Die vorliegende Erfindung betrifft eine Vorrichtung zum Zuführen von Wärme an eine Hautpartie eines Lebewesens. Die vorliegende Erfindung betrifft außerdem ein Infrarotheizmodul zum Zuführen von Wärme an eine Hautpartie eines Lebewesens. Die vorliegende Erfindung betrifft ferner eine erfindungsgemäße Vorrichtung, die ein erfindungsgemäßes Infrarotheizmodul aufweist.

Stand der Technik

Aus dem Stand der Technik sind Infrarotstrahler für den Einsatz bei Wärmeanwendungen bekannt. Allerdings ist es bei bekannten Strahlern häufig kompliziert, oder teils gar nicht möglich, die abgegebene Wärme zu kontrollieren. Dadurch führen Wärmeanwendungen nicht immer zu optimalen Ergebnissen.

Es ist daher eine Aufgabe der vorliegenden Erfindung, Mittel anzugeben, mit denen die Nachteile des Stands der Technik überwunden werden können und die insbesondere eine zuverlässige, effiziente und sichere Wärmeanwendung ermöglichen.

Beschreibung der Erfindung

Die Aufgabe wird durch die Erfindung gemäß einem **ersten Aspekt** dadurch gelöst, dass eine Vorrichtung zum Zuführen von Wärme an eine Hautpartie eines Lebewesens, wobei die Vorrichtung drei oder mehr als drei Infrarotheizmodule aufweist, und wobei vorzugsweise die Vorrichtung zumindest ein Abstützelement zum zumindest teilweisen Abstützen der Vorrichtung an dem Lebewesen aufweist, vorgeschlagen wird.

Dem liegt damit die überraschende Erkenntnis zugrunde, dass der Hautpartie die Wärmestrahlung besonders kontrolliert bereitgestellt werden kann, indem die Vorrichtung definiert oder definierbar relativ zu der Hautpartie anordenbar ist.

Dadurch ist es besonders zuverlässig möglich, einen definierten oder definierbaren Abstand zwischen dem einen, aber vor allem den mehreren, Infrarotheizmodulen und der Hautpartie vorzugeben. Dies wiederum ermöglicht es, eine abgestimmte Verteilung der von den Infrarotheizmodulen abgegebenen Infrarotstrahlung und/oder Wärme zwischen der Vorrichtung und der Hauptpartie zu ermöglichen und damit einen zuverlässigen Einsatz der Vorrichtung zu fördern. Außerdem wird der Einsatz der Vorrichtung durch den Anwender aufgrund des Abstützelements vereinfacht, da mittels des Abstützelements die Vorrichtung leichter in eine richtige Position gebracht und dort gehalten werden kann. Dadurch wird nicht zuletzt auch die Sicherheit beim Einsatz einer solchen Vorrichtung erhöht.

Das Lebewesen kann vorliegend beispielsweise ein Mensch oder ein Tier sein.

Die Hautpartie kann die Hautpartie einer Körperregion des Lebewesens sein. Beispielsweise kann es eine Hautpartie des Rückens oder des Beins, insbesondere des Knies, des Lebewesens sein.

Die Vorrichtung kann beispielsweise drei, vier, fünf, sechs, sieben, acht, neun, zehn oder mehr als zehn Infrarotheizmodule aufweisen. Durch das Bereitstellen mehrerer Infrarotheizmodule und die mittels des Abstützelements realisierbare, definierte relative Anordnung der Vorrichtung und des Lebewesens zueinander, kann eine optimale und gleichmäßige Bestrahlung des Lebewesens erreicht werden.

Alternativ oder ergänzend kann vorgesehen sein, dass die Vorrichtung wenigstens ein Gehäuse aufweist, und wobei vorzugsweise innerhalb eines von dem Gehäuse zumindest bereichsweise eingeschlossenen Volumens die Infrarotheizmodule angeordnet sind.

Das Gehäuse kann beispielsweise einstückig oder mehrteilig ausgeführt sein. Neben den darin angeordneten Infrarotheizmodulen ist auch weitere Elektronik innerhalb des von dem Gehäuse eingeschlossenen Volumens anordenbar. Damit sind gerade die elektrischen Komponenten der Vorrichtung sicher untergebracht.

Alternativ oder ergänzend kann vorgesehen sein, dass (i) das zumindest eine Abstützelement an einer Seitenpartie und/oder an einer Frontpartie des Gehäuses direkt oder indirekt angeordnet ist, (ii) mehrere gleichartige Abstützelemente an der Vorrichtung, insbesondere ihrer Seiten- und/oder Frontpartie, angeordnet sind und/oder (iii) das zumindest eine Abstützelement zum zumindest teilweisen Abstützen der Vorrichtung an einem Rückenbereich des Lebewesens geeignet ist.

Indem das Abstützelement an einer Seitenpartie des Gehäuses angeordnet ist, beispielsweise also seitlich von dem übrigen Teil der Vorrichtung herausragt, kann der mittlere Teil der Vorrichtung kompakter ausgestaltet werden. Ist hingegen das Abstützelement an einer Frontpartie des Gehäuses angeordnet, kann die Vorrichtung robuster ausgeführt werden und beispielsweise auch einen Teil des Rückens des Lebewesens komplett umschließen.

Das Abstützelement ist vorzugsweise dann direkt an der Partie des Gehäuses angeordnet, wenn kein weiteres Element zwischen Abstützelement und Gehäuse vorgesehen ist und ist anderenfalls indirekt an der Partie des Gehäuses angeordnet.

Es kann freilich vorzugsweise auch ganz allgemein vorgesehen sein, dass das zumindest eine Abstützelement an dem Gehäuse direkt oder indirekt angeordnet ist.

Alternativ oder ergänzend kann vorgesehen sein, dass das Abstützelement eine spezifische Oberfläche bereitstellt, die vorzugsweise zum Abstützen der Vorrichtung an dem Lebewesen mit einer Kontaktoberfläche, insbesondere eines Rückenbereichs, des Lebewesens in Kontakt bringbar ist, wobei der Verlauf und/oder die Gestalt der spezifischen Oberfläche zumindest abschnittsweise und/oder bereichsweise an den Verlauf und/oder die Gestalt der Kontaktoberfläche anpassbar ist.

Indem das Abstützelement also in dem Oberflächenbereich (nämlich derjenige der spezifischen Oberfläche), in dem es beim Einsatz der Vorrichtung an dem Lebewesen aufkommt, gewissermaßen an die Kontur des Lebewesens anschmiegbar ist, wird ein besonders komfortabler und sicherer Einsatz der Vorrichtung ermöglicht.

Alternativ oder ergänzend kann vorgesehen sein, dass das Abstützelement mit spezifischer Oberfläche mehrere, vorzugsweise gegeneinander bewegbare, insbesondere in ihrer Position und/oder Lage veränderbare, und/oder, insbesondere durch Komprimierung, in ihrer Form und/oder Gestalt reversibel veränderbare, Einzelelemente aufweist und jedes Einzelelement mit einem Oberflächenbereich einen Teil der spezifischen Oberfläche des Abstützelements bereitstellt.

Die Einzelelemente können damit jeweils einen individuellen Oberflächenbereich bereitstellen und alle Einzelelemente zusammen bilden dann die spezifische Oberfläche des Abstützelements.

Durch die Einzelelemente ist es besonders einfach möglich, den Verlauf der spezifischen Oberfläche bereichsweise/abschnittsweise an den Verlauf der Oberfläche des Lebewesens anzupassen. Denn die spezifische Oberfläche wird sozusagen durch mehrere kleinere Oberflächen zusammengestellt. Dadurch kann der lokale Verlauf der Oberfläche des Lebewesens (jedenfalls bereichsweise bei der Kontaktoberfläche) besser nachempfunden werden und somit der Sitz der Vorrichtung an dem Lebewesen verbessert werden.

Indem nämlich die einzelnen Einzelelemente gegeneinander bewegbar sind, kann der Verlauf und/oder die Gestalt der spezifischen Oberfläche anpassbar sein. Auch kann vorzugsweise der Oberflächenbereich in seiner Form und/oder Gestalt reversibel veränderbar sein.

Es ist bevorzugt, dass die spezifische Oberfläche vollständig durch die Oberflächenbereiche der Einzelelemente bereitgestellt wird. Dabei ist es möglich, dass die spezifische Oberfläche nicht zusammenhängend ist. Beispielsweise kann in dem Bereich zwischen den einzelnen Einzelelementen eine Unterbrechung der spezifischen Oberfläche auftreten.

Alternativ oder ergänzend kann vorgesehen sein, dass das Abstützelement mit spezifischer Oberfläche ein elastisches Grundelement, insbesondere bestehend aus oder aufweisend Silikon, aufweist, auf dem die Einzelelemente angeordnet sind, und insbesondere stoffschlüssig mit dem elastischen Grundelement verbunden sind.

Indem das elastische Grundelement vorgesehen ist, kann besonders einfach erreicht werden, dass die Einzelelemente gegeneinander bewegbar sind. Denn das Abstützelement ist in diesem Fall besonders gut dafür einsetzbar, dass durch eine äußere auf das Abstützelement einwirkende Belastung (durch das Lebewesen) die Einzelelemente abhängig von dem lokal bestehendem Belastungsdruck in das elastische Grundelement hineingedrückt werden, etwa indem das elastische Grundelement lokal komprimiert wird, und dadurch die Position und/oder Lage des Einzelelements verändert wird.

Das elastische Grundelement kann einstückig ausgebildet sein. Alternativ kann es auch mehrteilig ausgebildet sein. Dann können beispielsweise unterschiedliche Einzelelemente auf verschiedenen Teilen des elastischen Grundelements angeordnet sein.

Alternativ oder ergänzend kann vorgesehen sein, dass (i) die Einzelelemente, vorzugsweise nebeneinander und/oder flächig, auf dem elastischen Grundelement angeordnet sind, (ii) das elastische Grundelement schichtförmig ausgebildet ist, (iii) das elastische Grundelement, insbesondere unmittelbar, zwischen einerseits den Einzelelementen und andererseits dem Gehäuse angeordnet ist, vorzugsweise das elastische Grundelement direkt auf dem Gehäuse angeordnet ist, (iv) das elastische Grundelement quaderförmig ist und/oder (v) das elastische Grundelement eine Dicke von wenigstens 0,50 cm und/oder von maximal 10 cm, vorzugsweise maximal 5 cm, aufweist.

Einen besonders einfachen aber dennoch wirkungsvollen Aufbau kann mit dem schichtförmig ausgebildeten oder quaderförmigen elastischen Grundelement erreicht werden. Gleichermaßen ist ein einfacher und kostengünstiger Herstellungsprozess möglich, indem das elastische Grundelement direkt auf dem Gehäuse aufgebracht ist. Optional kann auch beispielsweise ein Haftvermittler zwischen dem elastischem Grundelement und dem Gehäuse vorgesehen sein.

Alternativ oder ergänzend kann vorgesehen sein, dass die Einzelelemente selbst nicht-kompressibel sind und/oder als Material Kunststoffe, Kunstleder, Leder und/oder sonstige Naturmaterialien aufweisen oder daraus bestehen.

Vor allem im Zusammenspiel mit einem elastischen Grundelement müssen die Einzelelemente nicht kompressibel sein, um eine Oberfläche des Abstützelements mit anpassbarem Verlauf und/oder anpassbarer Gestalt bereitzustellen. Dadurch kann der Vorteil einer anpassbaren Oberfläche auch für Einzelelementen erreicht werden, die mit kostengünstigen Verfahren, wie Spritzguss, hergestellt oder herstellbar sind.

Indem die Einzelelemente aus einem kompressiblen Material sind, können der Verlauf und/oder die Gestalt der spezifischen Oberfläche optimal an den Verlauf der Kontaktoberfläche angeglichen werden, wodurch ein besonders guter Halt der Vorrichtung an dem Lebewesen ermöglicht werden kann.

Alternativ oder ergänzend kann vorgesehen sein, dass das Abstützelement mit spezifischer Oberfläche 2 oder mehr als 2, vorzugsweise 5 oder mehr als 5, vorzugsweise 10 oder mehr als 10, und/oder 20 oder weniger als 20, vorzugsweise 15 oder weniger als 15, vorzugsweise 10 oder weniger als 10, vorzugsweise 7 oder weniger als 7, vorzugsweise 5 oder weniger als 5, vorzugsweise 3 oder weniger als 3, Einzelelemente aufweist.

Alternativ oder ergänzend kann vorgesehen sein, dass die Vorrichtung, insbesondere an der Seitenpartie und/oder an der Frontpartie, zumindest ein, vorzugsweise pyramidenstumpfförmige oder parallelepipedförmige, Podestelement aufweist, vorzugsweise mehrere solcher Podestelemente aufweist, und wobei vorzugsweise die Podestelemente von dem Gehäuse ausgebildet werden.

Ein solches Podestelement ermöglicht es besonders einfach, bestimmte Elemente von übrigen Teilen der Vorrichtung beabstandet und vor allem exponiert zu positionieren oder positionieren zu können.

Alternativ oder ergänzend kann vorgesehen sein, dass die Vorrichtung zumindest einen Infrarot-Temperatursensor aufweist, der insbesondere dazu eingerichtet ist, eine lokale Hauttemperatur des Lebewesens zu messen, wobei vorzugsweise der Sensor (i) an dem Gehäuse angeordnet ist, (ii) derart an der Vorrichtung, insbesondere an dem Gehäuse, angeordnet ist, dass der Sensor beim Einsatz der Vorrichtung zwischen den Infrarotheizmodulen und der Hautpartie des Lebewesens angeordnet ist, (iii) außerhalb des von dem Gehäuse zumindest bereichsweise eingeschlossenen Volumens angeordnet ist, (iv) im Bereich eines Podestelements angeordnet ist, (v) an einem Podestelement angeordnet ist, insbesondere an einer Seitenfläche des Podestelements, und/oder (vi) ganz oder teilweise innerhalb eines Podestelements angeordnet ist, und wobei vorzugsweise das Podestelement, insbesondere einer seiner Seitenflächen, zumindest bereichsweise einen für die Infrarot-Temperaturmessung durchlässigen Wandbereiche aufweist.

Mittels eines solchen Sensors kann die jeweils bestehende Hauttemperatur in einem von dem Sensor erfassten Hautbereich gemessen werden. Anhand des Temperaturwertes kann wiederum die von den Infrarotheizmodulen abgegebene Wärmestrahlung geregelt werden, so dass eine Überhitzung der Hauptpartie vermieden werden kann. Dadurch ist der Einsatz der Vorrichtung sicherer.

Indem der Sensor zwischen den Infrarotheizmodulen und der Hautpartie angeordnet ist, kann der Sensor einen ausreichenden Abstand von den Infrarotheizmodulen aufweisen, so dass die Messung nicht oder nicht erheblich von den Infrarotheizmodulen und der von diesen ausgestrahlten Infrarotstrahlung gestört wird.

Wenn davon gesprochen wird, dass der Sensor "zwischen" den Infrarotheizmodulen und der Hautpartie angeordnet ist, dann kann dies vorzugsweise auch den Fall mit einschließen, dass der Sensor lateral, also nach außen hin, versetzt angeordnet ist.

Durch die exponierte Anordnung des Sensors an oder in einem Podestelement kann erreicht werden, dass andere Teile der Vorrichtung die freie Sicht des Sensors auf die Hautpartie nicht beeinträchtigen oder gefährden.

Indem der Sensor an einem Podestelement angeordnet ist, lässt sich der Blickwinkel des Sensors und damit der mittels des Sensors überwachte Hautbereich besonders zuverlässig einstellen. Insbesondere das Vorsehen des Sensors an einer Seitenfläche eines Podestelements ermöglicht durch eine entsprechend geneigte Seitenfläche einen besonders vorteilhaften Blickwinkel auf die Hautpartie des Lebewesens. Indem der Sensor innerhalb eines Podestelements angeordnet ist, kann ein besonders kompaktes Design erreicht werden. Gerade in Verbindung mit einem für die Infrarot-Messung durchlässigen Wandbereich kann der Sensor komplett in der Vorrichtung verborgen werden, wodurch der Sensor vor externen Einflüssen wie Berührung oder Schweiß zuverlässig geschützt sein kann.

Der Wandbereich kann für die Infrarot-Temperaturmessung durchlässig sein, wenn beispielsweise der Wandbereich für Infrarotstrahlung im Bereich zwischen 0,78 $\mu$m und 15 $\mu$m einen Transmissionsgrad von 30 % oder mehr, vorzugsweise von 50 % oder mehr, bevorzugt von 80 % oder mehr, aufweist.

Alternativ oder ergänzend kann vorgesehen sein, dass das Abstützelement mit spezifischer Oberfläche, vorzugsweise alle Abstützelemente mit spezifischer Oberfläche, jeweils auf einem Podestelement angeordnet ist und/oder auf Silikon gelagert angeordnet ist.

Indem das Abstützelement auf einem Podestelement angeordnet ist, kann die Distanz zwischen dem Lebewesen und Teilen der Vorrichtung (beispielsweise den Infrarotheizmodulen) besonders einfach angepasst werden, ohne die Abstützelemente selbst ändern zu müssen. Mit anderen Worten, es kann mit nur einem Typ von Abstützelement verschiedene Abstände erreicht werden. Damit kann beispielsweise auch für Modelle der Vorrichtung mit unterschiedlichen Abmessungen und daher mit unterschiedlich notwendigen Abständen zu der Hautpartie, der Wärme zugeführt werden soll, die gleichen Abstützelemente eingesetzt werden. Die unterschiedlichen Abstände können über die Abmessungen der jeweiligen Podestelemente eingestellt werden. Dadurch sind die Produktionskosten der Vorrichtung geringer. Ein gerade bei großen Stückzahlen nicht zu unterschätzender wirtschaftlicher Faktor.

Indem das Abstützelement auf Silikon gelagert angeordnet ist, kann auf das Abstützelement ausgeübter Druck, beispielsweise bei Beanspruchung durch das Lebewesen, zumindest zum Teil abgefangen werden, ohne das Abstützelement zu beschädigen. Dadurch hält das Abstützelement auch höheren Belastungen besser Stand.

Alternativ oder ergänzend kann vorgesehen sein, dass das Abstützelement mit spezifischer Oberfläche eine Rückenlehne ausbildet.

Vorzugsweise sind mehrere Abstützelemente von der Vorrichtung aufgewiesen und mehr als eines, insbesondere alle, dieser Abstützelemente bilden die Rückenlehne aus.

Die Rückenlehne ermöglicht ein sicheres Anordnen der Vorrichtung an dem Lebewesen.

Alternativ oder ergänzend kann vorgesehen sein, dass das Abstützelement mit spezifischer Oberfläche zerstörungsfrei lösbar und/oder austauschbar an der Vorrichtung, insbesondere an dem Podestelement, angeordnet ist.

Dadurch sind beschädigte Abstützelemente leicht ersetzbar, was die Vorrichtung günstig im laufenden Unterhalt macht. Auch können dadurch unterschiedliche Abstützelemente angeboten werden (zum Beispiel hinsichtlich Größe, Steifigkeit, Material, etc.) aus denen abhängig vom jeweiligen Anwendungsszenario und/oder Benutzer ausgewählt werden kann. So lässt sich die Vorrichtung besonders einfach individuell konfigurieren und sicher einsetzen.

Alternativ oder ergänzend kann vorgesehen sein, dass der Oberflächenbereich zumindest eines, vorzugsweise mehrere, der Einzelelemente eines jeden Abstützelements mit spezifischer Oberfläche sechseckförmig ist.

Insbesondere eine wabenförmige Struktur ist dabei besonders vorteilhaft, da benachbarte Einzelelemente einen überlappenden Übergangsbereich bereitstellen können, wodurch die spezifische Oberfläche besonders zuverlässig in ihrem Verlauf anpassbar ist.

Alternativ oder ergänzend kann vorgesehen sein, dass die Vorrichtung einen Berührschutz aufweist, wodurch ein unbeabsichtigtes Berühren der Infrarotheizmodule verhinderbar ist, wobei der Berührschutz vorzugsweise in einer von dem Gehäuse ausgebildeten Öffnung angeordnet ist und/oder diese zumindest teilweise ausfüllt.

Durch den Berührschutz kann der Einsatz der Vorrichtung sicherer gestaltet sein, da ein versehentliches Berühren der Infrarotheizmodule verhindert oder zumindest erschwert ist.

Alternativ oder ergänzend kann vorgesehen sein, dass der Berührschutz gitterförmig ist, insbesondere mehrere zueinander beabstandete Gitterstäbe und/oder Saiten aufweist oder daraus besteht.

Ein derart gestalteter Berührschutz ermöglicht einen besonders effektiven Berührschutz, bei einem nur geringen Materialeinsatz. Dementsprechend kann dadurch auch das Gewicht der Vorrichtung reduziert sein, was wiederum auch das Handhaben und der Vorrichtung einfacher und sicherer gestaltet.

Alternativ oder ergänzend kann vorgesehen sein, dass die Vorrichtung eine Beleuchtung aufweist, wobei vorzugsweise die Beleuchtung zumindest einen seitlichen Innenteil des Gehäuses beleuchtet oder an diesem angeordnet ist.

Die Beleuchtung kann die Wartung der Vorrichtung besonders einfach und sicher gestalten. Aber auch der Einsatz der Vorrichtung lässt sich durch die Beleuchtung sicherer gestalten, indem beispielsweise die Beleuchtung dazu eingerichtet ist, einem Benutzer einen Zustand der Vorrichtung zu signalisieren. Beispielsweise kann durch Farbe, Rhythmus und/oder Frequenz der Beleuchtung ein bestimmter Zustand signalisiert werden.

Alternativ oder ergänzend kann vorgesehen sein, dass die Vorrichtung eine Kopfstütze und ein Haltemittel aufweist, wobei die Kopfstütze mittels des Haltemittels beabstandet zu dem Gehäuse der Vorrichtung befestigt oder befestigbar ist.

Die Kopfstütze ermöglicht einen angenehmen und sicheren Einsatz der Vorrichtung.

Alternativ oder ergänzend kann vorgesehen sein, dass die Kopfstütze eine Plastikschale und ein daran angeordnetes flexibles Netzgewebe aufweist.

Die Plastikschale einer derartigen Kopfstütze ist günstig beispielsweise mittels Spritzgusses herstellbar. Durch das Netzgewebe kann eine komfortable Abstützung des Kopfes des Benutzers der Vorrichtung erreicht werden. Das flexible Netzgewebe überspannt beispielsweise einen Öffnungsbereich der Plastikschale. Dadurch lässt sich das Netzgewebe durch Belastung durch den Kopf in den Öffnungsbereich hinein dehnen und passt sich damit der Kopfform des Benutzers an und vermittelt so eine zuverlässige Abstützung.

Alternativ oder ergänzend kann vorgesehen sein, dass das Haltemittel von dem Gehäuse lösbar und/oder in dem Gehäuse versenkbar ist und/oder federnde Eigenschaften hat.

Alternativ oder ergänzend kann vorgesehen sein, dass die Kopfstütze als Gehäuseabdeckung in einem beim Einsatz der Vorrichtung unteren und/oder oberen Bereich an dem Gehäuse lösbar befestigbar ist.

Dadurch lässt sich die Vorrichtung bei Nichtgebrauch kompakt aufbewahren.

Alternativ oder ergänzend kann vorgesehen sein, dass die Vorrichtung einen Aroma-Diffusor aufweist, insbesondere im beim Einsatz der Vorrichtung oberen Bereich der Vorrichtung.

Alternativ oder ergänzend kann vorgesehen sein, dass die Infrarotheizmodule jeweils ein lasergeschnittenes Heizelement oder ein Flächenheizelement aus Kunstglimmer aufweisen und/oder eine Abdeckung aus, insbesondere, eloxiertem, Aluminium aufweisen.

Dadurch kann gerade der Rückenbereich eines Lebewesens besonders gut mit Wärme versorgt werden.

Alternativ oder ergänzend kann vorgesehen sein, dass die Infrarotheizmodule beim Einsatz der Vorrichtung derart relativ zu und/oder an dem Lebewesen angeordnet sind, dass für eine definierte oder definierbare spezifische Transversalebene des Lebewesens jedes der Infrarotheizmodule eine Schnittfläche mit der spezifischen Transversalebene hat, wobei vorzugsweise jedes Infrarotheizmodul auf einer individuellen Betriebstemperatur betreibbar ist, und/oder

beim Einsatz der Vorrichtung die Betriebstemperaturen der Infrarotheizmodule derart zumindest teilweise unterschiedlich eingestellt oder einstellbar sind, dass die Intensität der, insbesondere beim Einsatz der Vorrichtung, von der Vorrichtung bereitgestellten Wärmestrahlung zum Zuführen an die Hautpartie zumindest entlang einer ersten und einer zweiten jeweils parallel zur spezifischen Transversalebene verlaufenden Richtung innerhalb zumindest eines betrachteten Bereichs außerhalb der Vorrichtung homogen ist.

Dieser Ausgestaltung liegt die überraschende Erkenntnis zugrunde, dass der Hautpartie die Wärmestrahlung besonders kontrolliert bereitgestellt werden kann, indem die Abstrahlcharakteristik der einzelnen Infrarotheizmodule berücksichtigt wird. Dadurch kann in einem bestimmten Bereich außerhalb des Strahlers ein auf die jeweilige Anwendung abgestimmtes Wärmefeld bereitgestellt werden. Wenn sich bei der Wärmebehandlung die Hautpartie innerhalb dieses Bereichs befindet, kann damit eine zuverlässige Wärmebehandlung durchgeführt werden. Außerdem kann so besonders einfach das Risiko einer lokalen Überhitzung der Hautpartie — sogenannte Hotspots — in Folge eines zu hohen Wärmestrahlungsbeitrags mehrerer Infrarotheizmodule zuverlässig verringert werden (ggf. in Kombination mit weiteren Maßnahmen, insbesondere die Steuerung betreffend). Gleichzeitig können ganz entsprechend auch Bereiche mit zu geringer Wärmestrahlungszufuhr vermieden werden, wodurch eine zuverlässige und gleichmäßige Wärmeanwendung ermöglicht wird.

Die Erfinder haben insoweit erkannt, dass die unterschiedlichen Abstrahlcharakteristiken besonders einfach aber dennoch wirkungsvoll dadurch berücksichtigt werden können, indem die Infrarotheizmodule mit unterschiedlicher Temperatur betrieben werden. Dadurch ist es überraschend einfach möglich, innerhalb des betrachteten Bereichs, der außerhalb der Vorrichtung liegt, eine homogene Intensität der Wärmestrahlung, die sich aus überlagerten Beiträgen auch mehrerer Infrarotheizmodule ergeben kann, bereitzustellen. Da mit unterschiedlichen Betriebstemperaturen regelmäßig auch eine veränderliche Abstrahlcharakteristik einhergeht, kann durch eine gezielt einstellbare oder eingestellte Betriebstemperatur daher eine homogene Intensität der Wärmestrahlung in dem betrachteten Bereich erreicht werden.

In dem betrachteten Bereich wird gerade ein Teil derjenigen von der Vorrichtung bereitgestellten Wärmestrahlung betrachtet, die zum Zuführen an die Hautpartie verwendbar ist. Dadurch adressiert die vorgeschlagene Vorrichtung auch tatsächlich gerade die Wärmestrahlung (indem diese Wärmestrahlung eine homogene Intensität aufweist), auf die es maßgeblich bei einer Wärmebehandlung ankommt.

Der betrachtete Bereich erstreckt sich dabei mitunter gemäß dem Verlauf der ersten und zweiten Richtungen und verläuft daher insbesondere innerhalb oder parallel zu der spezifischen Transversalebene.

Eine homogene Intensität der Wärmestrahlung in dem betrachteten Bereich erlaubt damit eine zuverlässige Behandlung der Hautpartie mit Wärme, da zum Beispiel durch die Betriebstemperaturen die Wärmezufuhr innerhalb bestimmter Grenzen gehalten werden kann.

Gelegentlich wird in dieser Anmeldung der Einfachheit halber auch von einem "homogenen Wärmefeld" gesprochen, das innerhalb des betrachteten Bereichs besteht. Damit ist dann die besagte homogene Intensität der Wärmestrahlung gemeint, sofern sich aus dem jeweiligen Zusammenhang nichts anderes ergibt.

Das homogene Wärmefeld ermöglicht dabei nicht nur eine, vor allem hinsichtlich der zulässigen Grenzwerte, besonders kontrollierte Wärmebehandlung. Zusätzlich erlaubt ein Bereich mit einem homogenen Wärmefeld auch eine Toleranz gegenüber einer relativen Bewegung von Vorrichtung und Hautpartie. Denn auch bei einer versehentlichen relativen Verschiebung oder einer ungenauen Positionierung der Vorrichtung relativ zur Hautpartie ist eine zuverlässige Wärmebehandlung durchführbar. Dadurch ist der Einsatz der Vorrichtung anwenderfreundlich und robust.

Durch die unterschiedlichen Betriebstemperaturen ist also eine derartige Überlagerung der Wärmestrahlungs-Beiträge der einzelnen Infrarotheizmodule erreicht oder erreichbar, so dass sich eine homogene Intensität der Wärmestrahlung in dem betrachteten Bereich einstellt.

Unter der Betriebstemperatur eines Infrarotheizmoduls wird dabei vorzugsweise diejenige Temperatur verstanden, mit der das Infrarotheizmodul beim Einsatz der Vorrichtung Wärmestrahlung, insbesondere in einer Richtung hin zur Hautpartie, abstrahlt.

Durch die Wahl der einzelnen Betriebstemperaturen ist die Intensität der von den einzelnen Infrarotheizmodulen innerhalb des betrachteten Bereichs bereitgestellten Wärmestrahlung anpassbar, insbesondere verringerbar oder vergrößerbar.

Beispielsweise kann durch ein Anpassen der Betriebstemperatur eines Infrarotheizmoduls die Wellenlänge des Strahlungsmaximums der von dem Infrarotheizmodul abgestrahlten Wärmestrahlung im Spektrum verschoben werden und damit auch der Anteil der im Infrarotbereich, vorzugsweise im nahen Infrarotbereich, abgegebenen Wärmestrahlung angepasst, insbesondere erhöht oder verringert, werden.

Indem die einzelnen Infrarotheizmodule auf ganz oder teilweise unterschiedlichen Betriebstemperaturen betrieben werden, lässt sich somit innerhalb des betrachteten Bereichs die beschriebene homogene Intensität der Wärmestrahlung, insbesondere bezogen auf die zu bestrahlende Hautpartie, einstellen. Beispielsweise kann auch der absolute Intensitätswert innerhalb des betrachteten Bereichs einstellbar sein, etwa indem die Betriebstemperaturen gemeinsam abgesenkt und/oder erhöht werden.

Die Intensität der Wärmestrahlung in dem betrachteten Bereich ist dabei vorzugsweise dann homogen, wenn der Streuungsparameter

$$\delta = 2\frac{Imax - Imin}{Imax + Imin},$$

worin $I_{min}$ der Minimalwert und $I_{max}$ der Maximalwert der Intensität der Wärmestrahlung innerhalb des betrachteten Bereichs ist, einen Wert von 1,9 oder kleiner, vorzugsweise von 1,7 oder kleiner, vorzugsweise von 1,5 oder kleiner, vorzugsweise von 1,3 oder kleiner, vorzugsweise von 1,0 oder kleiner, vorzugsweise von 0,7 oder kleiner, vorzugsweise von 0,5 oder kleiner, vorzugsweise von 0,3 oder kleiner, vorzugsweise von 0,1 oder kleiner, aufweist.

Mit anderen Worten liegt eine homogene Intensität der Wärmestrahlung dann vor, wenn innerhalb des betrachteten Bereichs, insbesondere entlang der ersten und zweiten Richtung, die Schwankung der Intensität der Wärmestrahlung entsprechend begrenzt ist.

Vorzugsweise ist beim Einsatz der Vorrichtung diese relativ zu und/oder an dem Lebewesen abgeordnet. Alternativ oder ergänzend sind beim Einsatz der Vorrichtung alle oder einige der Infrarotheizmodule mit einer Spannung beaufschlagt. Dadurch strahlen diese Wärmestrahlung gemäß der dann jeweils bestehenden Betriebstemperatur ab.

Die Betriebstemperatur eines einzelnen Infrarotheizmodules liegt vorzugsweise jeweils zwischen 100 °C und 400 °C, vorzugsweise zwischen 100 °C und 300 °C, vorzugsweise zwischen 150 °C und 300 °C, vorzugsweise zwischen 150 °C und 250 °C oder zwischen 200 °C und 300 °C.

Eine Transversalebene des Lebewesens steht vorzugsweise senkrecht auf der Längsachse des Lebewesens. Im Fall eines aufrechtstehenden Menschen teilt die Transversalebene den menschlichen Körper in einen unteren und einen oberen Anteil. Es gibt beliebig viele parallel zueinander verlaufende Transversalebenen. Eine davon kann als die spezifische Transversalebene definierbar sowie ausgewählt und damit definiert sein.

Es versteht sich von selbst, dass die spezifische Transversalebene nicht Teil der Vorrichtung ist, sondern Mittel zum Zweck, um die Anordnung der Infrarotheizmodule sowie die Richtungen, entlang derer innerhalb des betrachteten Bereichs ein homogenes Wärmefeld besteht, zu definieren.

In einer Ausführungsform ist die Maximal-Intensität der Wärmestrahlung in dem betrachteten Bereich 1000 W/m$^2$ oder geringer, vorzugsweise 800 W/m$^2$, vorzugsweise 600 W/m$^2$ oder geringer, vorzugsweise 500 W/m$^2$ oder geringer, vorzugsweise 350 W/m$^2$ oder geringer, vorzugsweise 300 W/m$^2$ oder geringer, vorzugsweise 200 W/m$^2$ oder geringer, vorzugsweise 150 W/m$^2$ oder geringer, vorzugsweise 100 W/cm$^2$ oder geringer. Optional ist die Minimal-Intensität der Wärmestrahlung in dem betrachteten Bereich 50 W/m$^2$ oder größer, vorzugsweise 100 W/m$^2$ oder größer, vorzugsweise 200 W/m$^2$ oder größer, vorzugsweise 400 W/m$^2$ oder größer, vorzugsweise 500 W/m$^2$ oder größer, vorzugsweise 700 W/m$^2$ oder größer.

In einer Ausführungsform ist die Maximal-Intensität der Wärmestrahlung in dem betrachteten Bereich 200 mW/cm$^2$ oder geringer, vorzugsweise 170 mW/cm$^2$, vorzugsweise 150 mW/cm$^2$ oder geringer, vorzugsweise 130 mW/cm$^2$ oder geringer, vorzugsweise 100 mW/cm$^2$ oder geringer, vorzugsweise 70 mW/cm$^2$ oder geringer, vorzugsweise 50 mW/cm$^2$ oder geringer. Optional ist die Minimal-Intensität der Wärmestrahlung in dem betrachteten Bereich 20 mW/cm$^2$ oder größer, vorzugsweise 30 mW/cm$^2$ oder größer, vorzugsweise 50 mW/cm$^2$ oder größer, vorzugsweise 70 mW/cm$^2$ oder größer, vorzugsweise 100 mW/cm$^2$ oder größer, vorzugsweise 130 mW/cm$^2$ oder größer, vorzugsweise 150 mW/cm$^2$ oder größer.

Die Intensität der Wärmestrahlung an unterschiedlichen Punkten in dem betrachteten Bereich, insbesondere deren Minimal- und Maximalwert in dem betrachteten Bereich, kann beispielsweise mit dem Messgerät Ophir Vega, Messsensor Ophir 12A-V1, und/oder auf folgende Weise ermittelt werden: Der Messsensor wird in einem Abstand von 1 cm bis 30 cm (bevorzugt sind 4 cm) in einem definierten Raster/Matrix vor der abstrahlenden Vorrichtung, insbesondere vor dem jeweiligen abstrahlenden Element, positioniert. Optional kann die abstrahlende Vorrichtung / Element auch mit einer konstanten Geschwindigkeit in dem definierten Abstand abgefahren werden. Der Messsensor kann die Daten kontinuierlich aufzeichnen und darstellen.

Alternativ oder ergänzend kann vorgesehen sein, dass sich die Infrarotheizmodule entlang einer zur spezifischen Transversalebene senkrecht verlaufenden Richtung streifenförmig erstrecken.

Solche Infrarotheizmodule sind zuverlässig im Betrieb und kostengünstig in der Herstellung.

Alternativ oder ergänzend kann vorgesehen sein, dass beim Einsatz der Vorrichtung die erste Richtung parallel zu einer definierten oder definierbaren Sagittalebene des Lebewesens ist und/oder die erste Richtung eine Abstandsrichtung von der Vorrichtung zu der Hautpartie ist, und sich vorzugsweise der betrachtete Bereich 1 cm oder mehr und/oder 30 cm oder weniger entlang der ersten Richtung erstreckt.

Indem sich der betrachtete Bereich in Abstandsrichtung erstreckt, schwankt die bereitgestellte Intensität der Wärmestrahlung in Abstandsrichtung nicht oder nur innerhalb tolerierbarer Grenzen. Dadurch ist die Vorrichtung robust im Einsatz und die Wärmebehandlung kann zuverlässig durchgeführt werden. Vor allem kann die Vorrichtung dadurch gleichermaßen gut und zuverlässig bei unterschiedlichen Anatomien des Körpers, insbesondere des Rückens eines Menschen, eingesetzt werden. Eine homogene Strahlungsintensität ist damit beispielsweise über einen weiten Bereich eines Hohlkreuzes bereitstellbar.

Die Sagittalebene steht dabei senkrecht auf der spezifischen Transversalebene. Im Fall eines aufrechtstehenden Menschen erstreckt sich eine Sagittalebene von oben nach unten und hinten nach vorne. Vorzugsweise wird vorliegend als Sagittalebene diejenige Ebene betrachtet, die den Menschen mittig in eine linke und eine rechte Hälfte teilt.

Vorzugsweise erstreckt sich der betrachtete Bereich 2 cm oder mehr, vorzugsweise 3 cm oder mehr, vorzugsweise 5 cm oder mehr, vorzugsweise 7 cm oder mehr, vorzugsweise 10 cm oder mehr, vorzugsweise 15 cm oder mehr, vorzugsweise 18 cm oder mehr, vorzugsweise 20 cm oder mehr, vorzugsweise 25 cm oder mehr, entlang der ersten Richtung.

Vorzugsweise erstreckt sich der betrachtete Bereich 25 cm oder weniger, vorzugsweise 20 cm oder weniger, vorzugsweise 15 cm oder weniger, vorzugsweise 10 cm oder weniger, vorzugsweise 7 cm oder weniger, vorzugsweise 5 cm oder weniger, entlang der ersten Richtung.

Beispielsweise kann sich der Betrachtete Bereich zwischen 5 cm und 20 cm entlang der ersten Richtung erstrecken.

Alternativ oder ergänzend kann vorgesehen sein, dass beim Einsatz der Vorrichtung die zweite Richtung eine Umfangsrichtung des die Hautpartie aufweisenden Körperteils oder Körperbereichs des Lebewesens ist und/oder sich der betrachtete Bereich 10 cm oder mehr und/oder 100 cm oder weniger entlang der zweiten Richtung erstreckt.

Indem sich der betrachtete Bereich in Umfangsrichtung erstreckt, kann die Wärmebehandlung zuverlässig durchgeführt werden. Denn die bereitgestellte Intensität der Wärmestrahlung schwankt in Umfangsrichtung nicht oder nur innerhalb tolerierbarer Grenzen. Dadurch ist die Vorrichtung robust im Einsatz.

Die Umfangsrichtung kann sich dabei also entlang der Hautpartie erstrecken.

Die Umfangsrichtung kann dabei auch gekrümmt verlaufen. Dies kann gerade bei einer Hautpartie, die zum Rückenbereich oder dem Kniebereich gehört der Fall sein.

Vorzugsweise erstreckt sich der betrachtete Bereich 15 cm oder mehr, vorzugsweise 20 cm oder mehr, vorzugsweise 25 cm oder mehr, vorzugsweise 30 cm oder mehr, vorzugsweise 40 cm oder mehr, vorzugsweise 50 cm oder mehr, vorzugsweise 60 cm oder mehr, vorzugsweise 70 cm oder mehr, vorzugsweise 70 cm oder mehr, entlang der zweiten Richtung.

Vorzugsweise erstreckt sich der betrachtete Bereich 90 cm oder weniger, vorzugsweise 80 cm oder weniger, vorzugsweise 70 cm oder weniger, vorzugsweise 60 cm oder weniger, vorzugsweise 50 cm oder weniger, vorzugsweise 40 cm oder weniger, vorzugsweise 30 cm oder weniger, entlang der zweiten Richtung.

Beispielsweise kann sich der betrachtete Bereich zwischen 15 cm und 50 cm entlang der zweiten Richtung erstrecken.

Alternativ oder ergänzend kann vorgesehen sein, dass der betrachtete Bereich ein Volumenbereich, vorzugsweise mit wenigstens 50 cm$^3$ und/oder mit höchstens 30000 cm$^3$, ist, und/oder der betrachtete Bereich beim Einsatz der Vorrichtung zumindest teilweise zwischen Vorrichtung und Hautpartie liegt.

Wenn der betrachtete Volumenbereich entsprechend dimensioniert ist, eignet sich die Vorrichtung besonders gut für eine Wärmebehandlung einer Hautpartie des Rückens oder im Kniebereich. Auf diese Weise können nämlich auch großflächigere Wärmebehandlungen sicher und zuverlässig durchgeführt werden, da in einem entsprechend großen Volumenbereich.

Der betrachtete Volumenbereich kann sich dann entlang der ersten und zweiten Richtung sowie einer dritten Richtung erstrecken. Beispielsweise stehen alle drei Richtungen senkrecht aufeinander. Die dritte Richtung kann beispielsweise parallel zu einer Sagittalebene des Lebewesens verlaufen.

Wenn beispielsweise die erste oder zweite Richtung gekrümmt verläuft, kann auch der Volumenbereich eine entsprechend gekrümmte Form aufweisen. Damit kann der Volumenbereich sehr gut an die Gegebenheiten angepasst werden und somit ist dann auch die Wärmestrahlung in einem für diese Gegebenheit sinnvollem Bereich homogen.

Beispielsweise kann ein Koordinatensystem, etwa ein kartesisches Koordinatensystem, betrachtet werden, dessen Nullpunkt mittig, beispielsweise am Schwerpunkt, in der Vorrichtung liegt. Dann kann vorzugsweise definiert sein, dass die erste Richtung (beispielsweise in Form einer Abstandsrichtung) entlang der x-Achse, die zweite Richtung entlang der y-Achse und die dritte Richtung entlang der z-Achse verläuft. Beim Einsatz der Vorrichtung erstreckt sich der betrachtete Bereich dann vorzugsweise (a) zumindest abschnittsweise zwischen den absoluten Werten von +1 cm und +35 cm, vorzugsweise von +1 cm und +20 cm, entlang der x-Achse, (b) zumindest abschnittsweise zwischen den absoluten Werten von +50 cm und -50 cm, vorzugsweise von +32,5 cm und -32,5 cm, entlang der y-Achse, und/oder (c) zumindest abschnittsweise zwischen den absoluten Werten von +20 cm und -20 cm, vorzugsweise von +10 cm und -10 cm, entlang der z-Achse.

Der betrachtete Bereich kann also einem Volumenbereich entsprechen, der sich entlang der x-Achse von +5 cm bis +20 cm erstreckt (also sozusagen "vor" der Vorrichtung und zwischen Hautpartie und Vorrichtung liegt, wenn die positive x-Achse in Richtung der Hautpartie zeigt), der sich entlang der y-Achse (symmetrisch) von +30 cm bis -30 cm erstreckt, und der sich entlang der z-Achse (symmetrisch) von -10 cm bis +10 cm erstreckt.

Die Ausführungen können selbstverständlich auch auf den Fall angewendet werden, dass der betrachtete Bereich zweidimensional ist, wobei dann eine Achse, etwa die z-Achse, des kartesischen Koordinatensystems unberücksichtigt bleibt.

Beispielsweise beginnt der betrachtete Bereich 1 cm vor dem äußersten Element der Vorrichtung.

Im Fall eines Volumenbereichs liegt eine homogene Intensität der Wärmestrahlung in diesem Bereich vorzugsweise dann vor, wenn innerhalb des betrachteten Bereichs, insbesondere entlang der ersten, der zweiten Richtung und der dritten Richtung, die Schwankung der Intensität der Wärmestrahlung entsprechend der oben genannten Ungleichung begrenzt ist.

Alternativ oder ergänzend kann vorgesehen sein, dass der betrachtete Bereich sich seitlich entlang zumindest zweier benachbarter Infrarotheizmodule zumindest teilweise erstreckt.

Der betrachtete Bereich kann sich dann beispielsweise seitlich zur Haupterstreckung der Vorrichtung erstrecken. Dadurch weist also mit anderen Worten der betrachtete Bereich auch zumindest einen Übergangsbereich entlang zumindest zweier Infrarotheizmodule auf. Der Übergangsbereich befindet sich also lateral zu (also neben) der Vorrichtung.

Das ist sehr vorteilhaft, da sich auch die Hautpartie entlang mehrere Infrarotheizmodule erstreckt.

Dadurch ist also auch in solch einem Übergangsbereich eine Schwankung der Intensität der Wärmestrahlung ausgeschlossen oder zumindest begrenzt. Dadurch kann die Vorrichtung besonders einfach aus mehreren einzelnen Modulen aufgebaut sein, ohne dass es zu lokalen Hotspots auf der Hautpartie kommt. Dies wiederum ermöglicht es, den Verlauf der Hautpartie durch mehrere Infrarotheizmodule besonders gut nachzubilden. Dadurch steigert sich die Effizienz der möglichen Wärmebehandlung.

Alternativ oder ergänzend kann vorgesehen sein, dass beim Einsatz der Vorrichtung die Betriebstemperatur der einzelnen Infrarotheizmodule jeweils eingestellt oder einstellbar ist auf zwischen 100 °C und 400 °C, vorzugsweise zwischen 100 °C und 300 °C, vorzugsweise zwischen 150 °C und 300 °C, vorzugsweise zwischen 150 °C und 250 °C oder zwischen 200 °C und 300 °C.

Beispielsweise kann sich die Betriebstemperatur der beiden, vorzugsweise in der spezifischen Transversalebene, seitlichen Infrarotheizmodule jeweils zwischen 5 °C und 100 °C, vorzugsweise zwischen 5 °C und 50 °C, vorzugsweise zwischen 5 °C und 30 °C, von der höchsten Betriebstemperatur der übrigen Infrarotheizmodule unterscheiden. Damit wird eine besonders homogene Wärmebehandlung erreicht.

Alternativ oder ergänzend kann vorgesehen sein, dass beim Einsatz der Vorrichtung die Betriebstemperatur der beiden, vorzugsweise in der spezifischen Transversalebene, seitlichen Infrarotheizmodule jeweils (a) bis zu 50 %, vorzugsweise bis zu 40 %, vorzugsweise bis zu 30 %, vorzugsweise bis zu 20 %, vorzugsweise bis zu 10 %, größer eingestellt oder einstellbar ist als die höchste Betriebstemperatur der übrigen Infrarotheizmodule und/oder (b) 1% oder mehr, vorzugsweise 5 % oder mehr, vorzugsweise 10 % oder mehr, größer eingestellt oder einstellbar ist als die höchste Betriebstemperatur der übrigen Infrarotheizmodule.

Auf diese Weise kann besonders zuverlässig ein homogenes Wärmefeld erhalten werden.

Es ist dabei bevorzugt, dass die äußersten Infrarotheizmodule die höchste Betriebstemperatur aller Infrarotheizmodule aufweisen und dass die Betriebstemperaturen der übrigen Infrarotheizmodule nach innen hin abnimmt.

Alternativ oder ergänzend kann vorgesehen sein, dass die Betriebstemperatur eines jeden Infrarotheizmoduls mittels jeweils eines Bedienelements, wie einem Schiebeschalter, einem Drehknopf, einem Druckschalter oder einem berührungsempfindlichen Bedienfeld, an dem jeweiligen Infrarotheizmodul einstellbar ist.

Mit anderen Worten kann jedes Infrarotheizmodul ein solches Bedienelement aufweisen. Dadurch kann die Betriebstemperatur eines jeden Infrarotheizmoduls individuell für sich einstellbar sein.

Beispielsweise kann über ein erfindungsgemäßes Bedienelement eine Betriebstemperatur-Stufe zwischen 1 und 5 für jedes Infrarotheizmodul einstellbar sein. Jede Stufe entspricht dabei beispielsweise einer Betriebstemperatur innerhalb eines bestimmten Temperaturbereichs, der beispielsweise von 100 °C bis 400 °C reicht.

Alternativ oder ergänzend kann vorgesehen sein, dass die Vorrichtung ein Kontrollmodul aufweist, das dazu eingerichtet ist, die Betriebstemperaturen der einzelnen Infrarotheizmodulen, vorzugsweise basierend auf zumindest einem von einem Anwender ausgewählten oder auswählbaren Betriebsmodus, zu kontrollieren, insbesondere zu regeln oder zu steuern.

Indem die Betriebstemperaturen durch ein entsprechend eingerichtetes Kontrollmodul kontrolliert werden, also insbesondere eingestellt werden, kann der Betrieb der Vorrichtung zuverlässiger gestaltet werden. Denn die Verantwortung für die richtige Einstellung der Betriebstemperaturen wird dem Benutzer abgenommen. Das ermöglicht nicht nur eine sichere Anwendung, sondern ist auch komfortabel für den Benutzer.

Beispielsweise kann der Benutzer einen Betriebsmodus der Wärmebehandlung auswählen, der für ihn eine leicht verständliche Bezeichnung hat, wie etwa "Schonend" oder "Anregend". Das Kontrollmodul ist dann dazu eingerichtet, ein damit verknüpftes Kontrollieren der Betriebstemperaturen durchzuführen. Also beispielsweise auf einer eher geringen absoluten Intensität, oder einer eher hohen absoluten Intensität der Wärmestrahlung innerhalb des betrachteten Bereichs.

Grundsätzlich, also egal auf welche Weise die Betriebstemperatur einstellbar ist, kann es in einer Ausführungsform vorteilhaft sein, dass die Betriebstemperatur der einzelnen Infrarotheizmodule jeweils zwischen 100 °C und 400 °C, vorzugsweise zwischen 100 °C und 300 °C, vorzugsweise zwischen 150 °C und 300 °C, vorzugsweise zwischen 150 °C und 250 °C oder zwischen 200 °C und 300 °C, liegt. Dadurch besteht ein ausreichend großer Spielraum, um zuverlässig ein homogenes Wärmefeld zu erreichen, und wobei gleichzeitig die absoluten Intensitäten innerhalb akzeptabler Grenzwerte einstellbar sind.

Alternativ oder ergänzend kann vorgesehen sein, dass das Kontrollmodul dazu eingerichtet ist, wenigstens die beiden beim Einsatz der Vorrichtung seitlichen Infrarotheizmodule auf einer gleichen Betriebstemperatur zu betreiben und/oder wenigstens zwei der Infrarotheizmodule mit einer unterschiedlichen Betriebstemperatur zu betreiben.

Alternativ oder ergänzend kann vorgesehen sein, dass das Kontrollmodul dazu eingerichtet ist, die beiden beim Einsatz der Vorrichtung seitlichen Infrarotheizmodule mit gleicher Betriebstemperatur zu betreiben, die größer ist als jede der Betriebstemperaturen, auf denen die übrigen Infrarotheizmodule betrieben werden.

Indem die seitlichen Infrarotheizmodule auf einer höheren Betriebstemperatur als die übrigen Infrarotheizmodule betrieben werden, kann ein Abfall der bereitgestellten Intensität der Wärmestrahlung zu den Seiten hin zuverlässig reduziert oder vermieden werden. Außerdem kann auf diese Weise im Übergangsbereich von einem vorletzten Infrarotheizmodul zu dem letzten, also seitlichen Infrarotheizmodul, überraschenderweise mit besonders einfachen Mitteln zuverlässig ein homogenes Wärmefeld bereitgestellt werden.

Dabei kann es vorteilhaft sein, wenn der betrachtete Bereich sich dann zumindest teilweise entlang zumindest eines der seitlichen Infrarotheizmodul, vorzugsweise beider seitlicher Infrarotheizmodule, erstreckt.

Die seitlichen Infrarotheizmodule können dabei die in der spezifischen Transversalebene äußersten Infrarotheizmodule sein.

Beispielsweise kann die Betriebstemperatur der beiden seitlichen Infrarotheizmodule bis zu 50 %, vorzugsweise bis zu 40 %, vorzugsweise bis zu 30 %, vorzugsweise bis zu 20 %, vorzugsweise bis zu 10 %, größer sein als die höchste Betriebstemperatur der übrigen Infrarotheizmodule. Optional ist die Betriebstemperatur der beiden seitlichen Infrarotheizmodule aber 5 % oder mehr, vorzugsweise 10 % oder mehr, größer als die höchste Betriebstemperatur der übrigen Infrarotheizmodule.

Alternativ oder ergänzend kann vorgesehen sein, dass die Vorrichtung einen oder mehrere Sensoren aufweist, die dazu eingerichtet sind, jeweils eine lokale Temperatur der Hautpartie zu messen und wobei das Kontrollmodul dazu eingerichtet ist, zumindest basierend auf den gemessenen Temperaturwerten die Betriebstemperaturen der einzelnen Infrarotheizmodule zu kontrollieren, insbesondere zu regeln oder zu steuern.

Indem die Betriebstemperaturen basierend auf der konkreten Hauttemperatur kontrolliert werden, lassen sich lokale Hotspots auf der Hautpartie zuverlässig vermeiden oder zumindest reduzieren.

Beispielsweise kann durch den Sensor ein, insbesondere ausreichend großer, vernarbter Bereich der Hautpartie erkannt werden und die Betriebstemperaturen der Infrarotheizmodule entsprechend angepasst werden, um eine zu hohe Belastung des vernarbten Hautbereichs zu vermeiden. So lässt sich damit etwa kontrolliert die Intensität der Wärmestrahlung und damit die der Haut zugeführten Wärme reduzieren. Dies kann mit den Temperatursensoren sehr zuverlässig erkannt werden, da vernarbte Hautpartien eine andere Temperatur aufweisen als intakte Hautpartien.

Vorzugsweise ist also das Kontrollmodul dazu eingerichtet, die bereitgestellte Intensität der Wärmestrahlung basierend auf der Temperatur des empfindlichsten Hautbereichs einzustellen.

Dadurch kann sich zwar die absolute Intensität der Wärmestrahlung in dem betrachteten Bereich insgesamt reduzieren, also auch für an sich intakte Bereiche der Hautpartie; Allerdings kann dies vorteilhaft sein, um geschädigte Hautpartien schonend mit Wärme behandeln zu können.

So kann das Kontrollmodul beispielsweise dazu eingerichtet sein, die Betriebstemperaturen der einzelnen Infrarotheizmodule derart zu kontrollieren, dass die gemessenen Temperaturen eine Mindest-Temperatur nicht unterschreiten und/oder eine Maximal-Temperatur nicht überschreiten. Die Temperaturen stammen dabei von dem oder den Sensoren. Die Vorrichtung kann dadurch im Einsatz noch zuverlässiger und sicherer gemacht werden.

Das homogene Wärmefeld im betrachteten Bereich führt dabei zu einer höheren Sicherheit, dass eine nur lokal gemessene Temperatur der Hauptpartie auch für andere Regionen der Hautpartie Gültigkeit hat und dadurch eine zuverlässige Wärmebehandlung durchführbar ist.

Beispielsweise kann der Sensor in einer Ausführungsform dazu eingerichtet sein, die Hauptpartie abzuscannen und dadurch vorzugsweise mehrere ortsaufgelöste Temperaturwerte bereitzustellen.

Die Sensoren können beispielsweise in Form von Infrarottemperatursensoren ausgebildet sein. Dabei kann jeder Sensor bei dem Einsatz der Vorrichtung ein bestimmtes Sichtfeld auf die Hautpartie haben. Die Sichtfelder einiger Sensoren können sich jeweils zumindest teilweise überlappen. Dadurch können die lokalen Temperaturen der Hautpartie zuverlässig gemessen werden.

Beispielsweise können die Sensoren zusammen einen, vorzugsweise zusammenhängenden, Bereich der Hautpartie von bis zu 600 mm in einer Richtung und von bis zu 180 mm in eine andere Richtung erfassen. Alternativ oder ergänzend können die Temperaturen auf der Hautpartie für Hautbereiche mit einer längsten Ausdehnung im sub-Zentimeterbereich, vorzugsweise im sub-Millimeterbereich, erfasst werden.

Vorzugsweise sind die Sensoren verschiebbar innerhalb der Vorrichtung vorgesehen. Dadurch kann besonders einfach der überwachte Hautbereich an den jeweiligen Anwendungsfall angepasst werden. Beispielsweise können die Sensoren vertikal und/oder horizontal verschiebbar sein.

Alternativ oder ergänzend kann vorgesehen sein, dass die Sensoren matrixförmig angeordnet sind.

Dadurch lässt sich die Hautpartie sozusagen flächig abrastern, da jeder Sensor die Temperatur eines bestimmten Bereichs der Hautpartie ermittelt. Vorzugsweise sind die Erfassungsbereiche benachbarter Sensoren zumindest teilweise überlappend. Dadurch kann eine vollständige Erfassung der Hautpartie erreicht werden.

Anstelle einiger oder sogar aller der matrixförmig angeordneten Sensoren könnte auch ein 2D-Sensor vorgesehen sein, vorzugsweise weist dieser dann eine entsprechend große Auflösung auf. Mit anderen Worten, der 2D-Sensor könnte alternativ oder ergänzend zu matrixförmig angeordneten Sensoren vorgesehen sein. Es können aber auch mehrere 2D-Sensoren matrixförmig angeordnet sein.

Alternativ oder ergänzend kann vorgesehen sein, dass wenigstens zwei der Infrarotheizmodule relativ zueinander verschiebbar sind, insbesondere in einer Richtung, die beim Einsatz der Vorrichtung parallel zu der spezifischen Transversalebene verläuft.

Dadurch kann die Vorrichtung für verschiedene Körperbereiche des Lebewesens verwendet werden, da die Module leicht anpassbar sind. Auch ist es dadurch möglich, je nach Körperstatur, die Vorrichtung in eine passende Konfiguration zu überführen.

Optional kann die Vorrichtung zumindest ein Führungselement aufweisen, mittels dessen die Einheiten relativ zueinander veränderlich positionierbar sind, insbesondere (a) das Führungselement ein Teleskopauszug aufweist und die Einheiten an unterschiedlichen Segmenten des Teleskopauszugs angeordnet sind und/oder (b) das Führungselement eine Schiene aufweist, entlang derer die Einheiten zumindest abschnittsweise bewegbar und/oder mittels einer Rast-, Klemm-, und/oder Reibverbindung arretierbar sind.

Alternativ oder ergänzend kann vorgesehen sein, dass wenigstens zwei, vorzugsweise drei oder mehr als drei, der Infrarotheizmodule relativ zueinander neigbar sind.

Alternativ oder ergänzend kann vorgesehen sein, dass wenigstens zwei, vorzugsweise drei oder mehr als drei, der Infrarotheizmodule relativ zueinander abwinkelbar sind.

Die Möglichkeit, Infrarotheizmodule gegeneinander zu neigen oder abzuwinkeln ist gerade für die Wärmebehandlung von gekrümmten Hautpartien, wie auf dem Rücken oder am Knie, besonders vorteilhaft.

Der Krümmungswinkel liegt dabei vorzugsweise in der spezifischen Transversalebene.

Alternativ oder ergänzend kann vorgesehen sein, dass wenigstens zwei benachbarte Infrarotheizmodule, vorzugsweise alle paarweise benachbarten Infrarotheizmodule, thermisch voneinander entkoppelt sind, vorzugsweise durch einen Luftspalt, ein thermisches Isoliermaterial, und/oder ein Aerogel, das beispielsweise zwischen den Infrarotheizmodulen zumindest bereichsweise angeordnet ist.

Durch die Entkopplung kann ein Wärmefluss zwischen benachbarten Infrarotheizmodulen vermieden oder zumindest reduziert werden. Dadurch kann das homogene Wärmefeld, also eine homogene Intensität der Wärmestrahlung, noch besser und gezielter innerhalb des betrachteten Bereichs ausgebildet werden, da störende Einflüsse durch einen Wärmefluss zwischen den Modulen nicht oder in nicht mehr nennenswertem Ausmaß bestehen und ein solcher Wärmefluss daher nicht näher berücksichtigt werden muss.

Die Wärmeleitfähigkeit des thermischen Entkopplungsmittels beträgt vorzugsweise 0.05 W / mK oder weniger, vorzugsweise 0.03 W / mK oder weniger, vorzugsweise 0.02 W / mK oder weniger, vorzugsweise 0.01 W / mK oder weniger.

Alternativ oder ergänzend kann vorgesehen sein, dass jedes Infrarotheizmodul zumindest ein Infrarotstrahlerelement, insbesondere in Form eines Heizdrahtes, einer Folienheizung, eines Heizgewebes, von Mikanit, einer Dickschichtheizung und/oder einer Silikonheizmatte, aufweist oder darstellt. Insbesondere kann jedes Infrarotheizmodul zumindest ein Infrarotstrahlerelement in Form eines Carbongewebes aufweisen.

Ein Infrarotstrahlerelement ist vorliegend vorzugsweise ein Infrarotstrahler oder weist diesen auf. Ein Heizdraht ist ein günstiger und gleichzeitig robuster und zuverlässiger Infrarotstrahler. Ein Beispiel für eine Folienheizung ist eine Infrarot Wärmefolie.

Ein Infrarotstrahlerelement kann ein Teil mit unterschiedlichen Energiezonen sein oder mehrere Teile mit gleicher oder unterschiedlicher Leistung. Beispielsweise ist ein Infrarotstrahlerelement mit einer Elektronik ansteuerbar oder angesteuert und/oder oder jede Zone ist einzeln angesteuert oder ansteuerbar.

Je nach erforderlicher Betriebstemperatur können unterschiedliche Realisierungen bevorzugt sein. Eine Silikonheizmatte kann für Temperaturen bis 250 °C bevorzugt sein. Eine Folienheizung kann für Temperaturen bis 150 °C bevorzugt sein. Mikanit kann für Temperaturen bis 450 °C bevorzugt sein.

Ein Infrarotstrahlerelement kann dabei eine Ausgangs-Wärmestrahlung des jeweiligen Infrarotheizmoduls bereitstellen. Die Ausgangs-Wärmestrahlung kann qualitativ und/oder quantitativ identisch zu der Wärmestrahlung sein, die das Infrarotheizmodul seinerseits abstrahlt. Dies kann beispielsweise der Fall sein, wenn das Infrarotheizmodul identisch zu dem Infrarotstrahlerelement ist. Die Ausgangs-Wärmestrahlung kann auch in einem oder mehreren qualitativen oder quantitativen Punkten unterschiedlich zu der Wärmestrahlung sein, mit der das Infrarotheizmodul seinerseits abstrahlt. Dies kann beispielsweise der Fall sein, wenn das Infrarotheizmodul die von dem Infrarotstrahlerelement bereitgestellte Ausgangs-Wärmestrahlung modifiziert, etwa mittels Filter, Schirmungen, Isolierungen und/oder anderer Mechanismen wie ein durch die Wärmestrahlung aufwärmbares Element, das dann seinerseits Wärmestrahlung abstrahlt. Dieses Modifizieren kann ein Umlenken der Wärmestrahlung, ein Abschwächen der Wärmestrahlung und/oder ein Filtern von spektralen Anteilen der Wärmestrahlung aufweisen. Alternativ oder ergänzend kann ein Infrarotstrahlerelement eine Ausgangs-Wärmequelle des jeweiligen Infrarotheizmoduls darstellen. Das Infrarotstrahlerelement kann dann ein anderes Element des Infrarotheizmoduls aufwärmen, indem Wärme durch Konduktion von dem Infrarotstrahlerelement auf das andere Element übertragbar ist und/oder übertragen wird. Das andere Element kann dann beispielsweise seinerseits Wärmestrahlung abstrahlen und damit die von dem Infrarotheizmodul ausgestrahlte Wärmestrahlung ganz oder teilweise bereitstellen und/oder beeinflussen.

Indem das Infrarotheizmodul seinerseits ein Infrarotstrahlerelement aufweist, kann also vorteilhafterweise die von dem Infrarotheizmodul bereitgestellte, insbesondere an die Hautpartie zugeführte, Wärmestrahlung ausgehend von der Ausgangs-Wärmestrahlung und/oder von der Ausgangs-Wärmequelle des Infrarotstrahlerelement einstellbar sein.

Das Infrarotstrahlerelement stellt daher in einer Ausführungsform zumindest einen Teil der von dem Infrarotheizmodul abgestrahlten, und insbesondere an die Hautpartie zugeführten, Wärmestrahlung direkt oder indirekt (zum Beispiel durch Wärmestrahlung oder Konduktion) bereit.

In einer Ausführungsform ist das Infrarotheizmodul aber identisch zu dem Infrarotstrahlerelement. Dadurch ist die Vorrichtung kompakt im Aufbau.

Im Fall eines Heizdrahtes ist der Heizdraht vorzugsweise auf einem Trägermaterial, wie beispielsweise eine Textilie, angeordnet.

Es kann aber auch vorteilhaft sein, wenn das Infrarotstrahlerelement symmetrisch um die Mittelachse des Infrarotheizmoduls angeordnet ist. Besonders bevorzugt ist es dann, wenn die Temperatur, mit der das Infrarotstrahlerelement abstrahlt, entlang der Mittelachse die geringste Temperatur aufweist, die nach außen hin zunimmt.

Beispielsweise kann jedes Infrarotheizmodul zwei, drei, vier, fünf, sechs, sieben, acht, neun, zehn oder mehr als zehn Infrarotstrahlerelemente aufweisen. Dabei kann jedes Infrarotheizmodul die gleiche Anzahl Infrarotstrahlerelemente aufweisen, oder jedes Infrarotheizmodul kann eine individuelle Anzahl, insbesondere gemäß den vorstehenden Zahlenwerten, Infrarotstrahlerelemente aufweisen.

Beispielsweise können drei Infrarotheizmodule vorgesehen sein und jedes davon weist ein oder mehrere Infrarotstrahlerelemente auf. Die Infrarotstrahlerelemente der beiden seitlichen Infrarotheizmodule werden derart betrieben, dass die Infrarotheizmodule auf der gleichen Betriebstemperatur betrieben werden. Diese kann beispielsweise 250 °C betragen. Das oder die Infrarotstrahlerelemente des mittleren Infrarotheizmoduls wird derart betrieben, dass das Infrarotheizmodul auf einer kleineren Betriebstemperatur betrieben wird als die übrigen beiden. Diese kann beispielsweise 200 °C betragen.

Alternativ oder ergänzend kann vorgesehen sein, dass jedes Infrarotheizmodul ein Gehäuse oder Gehäuseabschnitt aufweist, innerhalb dessen vorzugsweise das jeweilige Infrarotstrahlerelement wie zuvor beschrieben angeordnet ist.

Das Gehäuse macht die Vorrichtung robust und damit sicher im Einsatz und ermöglicht ein sicheres Unterbringen aller, auch elektrischen, Komponenten.

Das Gehäuse kann eine Öffnung oder einen Bereich aufweisen, von der bzw. von dem ausgehend Wärmestrahlung in Richtung der Hautpartie abstrahlbar ist und/oder beim Einsatz der Vorrichtung abgestrahlt wird. Beim Einsatz der Vorrichtung ist dann vorzugsweise diese Öffnung oder dieser Bereich zumindest bereichsweise vor der Hautpartie angeordnet.

Das Gehäuse kann beispielsweise PU-Schaum aufweisen oder daraus bestehen. In dem Gehäuse können Sensoren und andere Elektronik integriert sein. Das Gehäuse kann auch einen Schweißablauf aufweisen.

In einer Ausführungsform ist eine thermische Isolierung zwischen dem Infrarotstrahlerelement und einem Abschnitt des Gehäuses vorgesehen. Beispielsweise kann hierzu ein Aerogel, insbesondere eine Schicht aus Aerogel, vorgesehen sein. Die thermische Isolierung, insbesondere die Aerogelschicht, kann eine Dicke von 5-20 mm, beispielsweise 10 mm, aufweisen. Durch die thermische Isolierung kann das Aufwärmen des Gehäuses vermieden oder zumindest reduziert werden. Dadurch wird die Effizient des Infrarotheizmoduls gesteigert sowie die Handhabung sicherer gestaltet.

Alternativ oder ergänzend kann vorgesehen sein, dass die Vorrichtung ein Gehäuse aufweist, innerhalb dessen die Infrarotheizmodule angeordnet sind.

Das Gehäuse der gesamten Vorrichtung macht die Vorrichtung robust und damit sicher im Einsatz.

Die Aufgabe wird durch die Erfindung gemäß einem **zweiten Aspekt** dadurch gelöst, dass ein Infrarotheizmodul zum Zuführen von Wärme an eine Hautpartie eines Lebewesens, aufweisend ein Gehäuse, zumindest ein innerhalb des Gehäuses angeordnetes Infrarotstrahlerelement, zumindest ein Arretierungsmittel und ein mit dem Arretierungsmittel lösbar in und/oder an dem Gehäuse und/oder an dem Infrarotstrahlerelement anordenbares Abstrahlelement, vorgeschlagen wird.

Der Erfindung liegt damit die überraschende Erkenntnis zugrunde, dass der Hautpartie die Wärmestrahlung besonders kontrolliert bereitgestellt werden kann, indem die Abstrahlcharakteristik des Infrarotheizmoduls passend eingestellt wird. Dadurch kann zuverlässig eine definierte Wärmebehandlung der Hautpartie erreicht werden, im Einklang mit der gewünschten Anwendung. Dadurch wird eine verbesserte Wärmeanwendung ermöglicht.

Die Erfinder haben insoweit erkannt, dass eine anpassbare Abstrahlcharakteristik des Infrarotheizmoduls besonders einfach dadurch erreicht werden kann, indem ein Abstrahlelement vorgesehen ist. Dieses kann die von dem Infrarotstrahlerelement bereitgestellte Wärmestrahlung und/oder die von dem Infrarotstrahlerelement auf das Abstrahlelement per Konduktion übertragene Wärme beispielsweise aufnehmen, insbesondere, indem es durch diese Strahlung und/oder Wärme erwärmt wird, und anschließend seinerseits Wärmestrahlung abstrahlen. Dadurch ist mittels des Abstrahlelements die Abstrahlcharakteristik des Infrarotheizmoduls anpassbar und/oder einstellbar.

Damit ist durch das Infrarotheizmodul eine Wärmestrahlung bereitstellbar, die beispielsweise auch hinsichtlich einer möglichst homogenen Intensität innerhalb eines Bereichs, wie einem Volumenbereich, der beim Einsatz des Infrarotheizmoduls zumindest teilweise zwischen Infrarotheizmodul und Hautpartie ist, einstellbar ist.

So kann, je nach Anwendungsfall, ein Abstrahlelement vorgesehen werden, das zu einer jeweils passenden Wärmestrahlung führt. Beispielsweise kann die Wärmestrahlung hinsichtlich Abstrahlrichtung und/oder Intensität innerhalb eines Bereichs, der vorzugsweise beim Einsatz des Infrarotheizmoduls zwischen Infrarotheizmodul und Hautpartie liegt, durch das Abstrahlelement veränderlich sein.

Da das Abstrahlelement lösbar an dem Gehäuse angeordnet ist, lässt sich das Abstrahlelement besonders einfach und ohne großen Aufwand austauschen und so die Abstrahlcharakteristik an einen neuen Anwendungsfall anpassen. Je nach Wahl des Arretierungsmittels ist dies sogar ohne zusätzliches Werkzeug möglich, wodurch die Handhabung sehr einfach ist. Das Infrarotheizmodul lässt sich dadurch schnell für einen neuen Anwendungsfall umrüsten.

Daher ist vorzugsweise das Abstrahlelement mit dem Arretierungsmittel ohne zusätzliches Werkzeug in und/oder an dem Gehäuse anordenbar.

Das Abstrahlelement erlaubt es auch, den Einfluss des konkret vorgesehenen Infrarotstrahlerelements und/oder der konkreten Anordnung des Infrarotstrahlerelements innerhalb des Gehäuses zu reduzieren. Denn das Abstrahlelement kann die Charakteristiken des Infrarotstrahlerelements anpassen. Beispielsweise kann ein linienförmiges Infrarotstrahlerelement eingesetzt werden, für das mittels des Abstrahlelements sehr einfach ein eher flächiges Abstrahlen erreicht werden kann. Dies ermöglicht es, dass in die Wahl des Infrarotstrahlerelements sowie dessen Anordnung innerhalb des Gehäuses auch andere Kriterien als die zur Erreichung einer konkreten Abstrahlcharakteristik fließen können. Dadurch können beispielsweise günstigere Elemente ausgewählt werden.

Das Abstrahlelement kann beispielsweise aus einem Metall, wie etwa Aluminium, sein. Dadurch ist eine besonders gute Wärmeleitfähigkeit gegeben, so dass das Abstrahlelement gut erwärmbar ist. Optional weist das Abstrahlelement ein Ofenlack und/oder eine Eloxalschicht auf oder ist damit zumindest bereichsweise beschichtet.

Vorzugsweise liegt beim Einsatz des Infrarotheizmoduls und von der Hautpartie aus betrachtet das Infrarotstrahlerelement hinter dem Abstrahlelement.

Vorzugsweise erstreckt sich das Abstrahlelement entlang seiner Haupterstreckungsrichtung 5 cm oder mehr, vorzugsweise 10 cm oder mehr, vorzugsweise 15 cm oder mehr, vorzugsweise 20 cm oder mehr, vorzugsweise 25 cm oder mehr, vorzugsweise 30 cm oder mehr, vorzugsweise 40 cm oder mehr, vorzugsweise 50 cm oder mehr, vorzugsweise 60 cm oder mehr, vorzugsweise 70 cm oder mehr, vorzugsweise 70 cm oder mehr.

Vorzugsweise erstreckt sich das Abstrahlelement entlang seiner Haupterstreckungsrichtung 90 cm oder weniger, vorzugsweise 80 cm oder weniger, vorzugsweise 70 cm oder weniger, vorzugsweise 60 cm oder weniger, vorzugsweise 50 cm oder weniger, vorzugsweise 40 cm oder weniger, vorzugsweise 30 cm oder weniger, vorzugsweise 20 cm oder weniger, vorzugsweise 15 cm oder weniger, vorzugsweise 10 cm oder weniger.

Beispielsweise hat das Abstrahlelement entlang seiner Haupterstreckungsrichtung eine Abmessung von zwischen 5 cm und 40 cm.

Außerdem ist einer oder mehrere der folgenden Aspekte besonders vorteilhaft:

- Das Abstrahlelement ist derart innerhalb des Gehäuses angeordnet, dass es von dem Infrarotstrahlerelement, insbesondere durch dessen beim Einsatz des Infrarotstrahlerelements abgestrahlte Wärmestrahlung und/oder von diesem per Konduktion übertragene Wärme, aufwärmbar ist. Dadurch kann es anschließend wieder Wärmestrahlung abgeben.

- Das Abstrahlelement ist zumindest teilweise vor, innerhalb und/oder hinter einer Öffnung des Gehäuses angeordnet und/oder verschließt eine Öffnung des Gehäuses zumindest teilweise. Dadurch kann das Abstrahlelement sozusagen "Innen" von "Außen" trennen.

- Das Abstrahlelement ist zumindest bereichsweise membranförmig und/oder flächig ausgebildet.

Vorzugsweise ist beim Einsatz des Infrarotheizmoduls dieses relativ zu und/oder an dem Lebewesen abgeordnet. Alternativ oder ergänzend ist beim Einsatz des Infrarotheizmoduls das Infrarotstrahlerelement mit einer Spannung beaufschlagt. Dadurch strahlt dieses Wärmestrahlung ab, insbesondere gemäß der jeweils bestehenden Betriebstemperatur des Infrarotstrahlerelements.

Es kann besonders bevorzugt sein, wenn das Infrarotstrahlerelement Merkmale des in Bezug auf den ersten Aspekt der Erfindung beschriebenen Infrarotstrahlerelements aufweist.

Alternativ oder ergänzend kann vorgesehen sein, dass beim Einsatz des Infrarotheizmoduls das Abstrahlelement zumindest bereichsweise zwischen dem Infrarotstrahlerelement und der Hautpartie angeordnet ist.

Beispielsweise kann dazu das Abstrahlelement zumindest teilweise vor, innerhalb und/oder hinter einer Öffnung des Gehäuses angeordnet und/oder das Abstrahlelement eine Öffnung des Gehäuses zumindest teilweise verschließen.

Alternativ oder ergänzend kann vorgesehen sein, dass das Arretierungsmittel in Form eines Schnellspannrahmens ausgebildet ist und vorzugsweise der Schnellspannrahmen mit dem Gehäuse verliersicher verbunden ist und/oder von dem Gehäuse bereitgestellt ist.

Mit einem Schnellspannrahmen kann das Abstrahlelement sehr schnell und einfach gewechselt werden.

Beispielsweise kann das Abstrahlelement dazu innerhalb eines Gehäusebereichs, der als Schnellspannrahmen fungiert, eingespannt werden. Mit anderen Worten, das Abstrahlelement kann von außen, insbesondere durch die Öffnung, innerhalb des Gehäuses anordenbar sein, indem es an einer vorgesehenen Stelle innerhalb des Gehäuses einspannbar ist. Dadurch ist das Abstrahlelement sehr einfach und ohne Werkzeug auswechselbar.

Beispielsweise kann der Schnellspannrahmen mehrteilig ausgeführt sein und vorzugsweise ein oder mehrere Komponenten des Infrarotheizmoduls zumindest bereichsweise sandwichartig zwischen den einzelnen Teilen des Schnellspannrahmens einspannen.

Der Schnellspannrahmen kann beispielsweise aus Blech sein oder ein Blech aufweisen.

Alternativ oder ergänzend kann vorgesehen sein, dass beim Einsatz des Infrarotheizmoduls das Abstrahlelement und das Infrarotstrahlerelement derart zueinander angeordnet sind, dass das Abstrahlelement durch das Infrarotstrahlerelement aufgewärmt wird und dann seinerseits Wärmestrahlung abgibt, insbesondere zumindest teilweise in Richtung der Hautpartie beim Einsatz des Infrarotheizmoduls, wobei vorzugsweise das Aufwärmen des Abstrahlelements zumindest teilweise durch zumindest einen Teil der von dem Infrarotstrahlerelement abgestrahlten Wärmestrahlung und/oder durch Konduktion von dem Infrarotstrahlerelement auf das Abstrahlelement erfolgt.

Indem das Abstrahlelement also eine gewissermaßen vermittelnde Stellung zwischen "Innen" und "Außen" einnimmt, ist die von dem Infrarotheizmodul, insbesondere in Richtung der Hautpartie, abgegebene Wärmestrahlung überraschenderweise besonders einfach und dennoch wirkungsvoll beeinflussbar.

Das Abstrahlelement ist also beispielsweise durch die Wärmestrahlung des Infrarotstrahlerelements und/oder durch per Konduktion von dem Infrarotstrahlerelement auf das Abstrahlelement übertragene Wärme erwärmbar. Wenn das Abstrahlelement erwärmt ist, strahlt es dann seinerseits Wärmestrahlung ab.

Alternativ oder ergänzend kann vorgesehen sein, dass das Arretierungsmittel von einer Entnahmestellung, in der vorzugsweise das Abstrahlelement von dem Gehäuse lösbar ist, in eine Arretierstellung, in der vorzugsweise das Abstrahlelement form-, reib- und/oder kraftschlüssig an dem Gehäuse und/oder an dem Infrarotstrahlerelement angeordnet ist und/oder mit dem Infrarotstrahlerelement zumindest bereichsweise in Kontakt steht, und umgekehrt überführbar ist.

Durch die definierten Stellungen des Arretierungsmittels ist eine sichere Handhabung des Infrarotheizmoduls möglich.

Ein besonders guter konduktiver Wärmeübertrag zwischen Infrarotstrahlerelement und Abstrahlelement ist möglich, indem diese in Kontakt stehen. Ein indirekter Kontakt kann dabei beispielsweise bestehen, wenn zwischen Infrarotstrahlerelement und Abstrahlelement beispielsweise eine Schutzschicht oder ein Verbindungsmittel vorgesehen ist. Beispiele hierfür ist eine thermisch leitende Schicht.

Alternativ oder ergänzend kann vorgesehen sein, dass das Arretierungsmittel eine Anlagefläche aufweist, die mit einem Oberflächenbereich des Abstrahlelements in Kontakt bringbar ist und wobei vorzugsweise das Infrarotheizmodul dazu ausgebildet ist, dass, wenn das Arretierungsmittel von der Entnahmestellung in die Arretierstellung überführt wird, das Abstrahlelement durch die mit ihm in Kontakt stehende Anlagefläche gegen das Gehäuse und/oder das Infrarotstrahlerelement gedrückt und dadurch das Abstrahlelement, während sich das Arretierungsmittel in der Arretierstellung befindet, form-, reib- und/oder kraftschlüssig an dem Gehäuse und/oder dem Infrarotstrahlerelement gehalten und/oder in direkten oder indirekten Kontakt mit dem Infrarotstrahlerelement gebracht wird.

Vorzugsweise ist das Abstrahlelement also zumindest bereichsweise sandwichartig zwischen einem Gehäuseteil und dem Arretierungsmittel gehalten, insbesondere wenn das Arretierungsmitel in der Arretierstellung ist. Alternativ oder ergänzend ist das Abstrahlelement zumindest bereichsweise sandwichartig zwischen dem Infrarotstrahlerelement und dem Arretierungsmittel gehalten, insbesondere wenn das Arretierungsmitel in der Arretierstellung ist, wobei vorzugsweise das Abstrahlelement und das Infrarotstrahlerelement in unmittelbarem Kontakt miteinander stehen.

Alternativ oder ergänzend kann vorgesehen sein, dass das Abstrahlelement scheibenförmig, insbesondere in runder oder eckiger Form, ausgebildet ist.

Dies lässt sich besonders einfach herstellen und zudem auch gut handhaben.

Alternativ oder ergänzend kann vorgesehen sein, dass das Gehäuse eine Öffnung mit einem, vorzugsweise ringförmigen, Randbereich aufweist, und vorzugsweise das Abstrahlelement an dem Randbereich angeordnet ist, insbesondere wenn das Arretierungsmittel in der Arretierstellung ist.

Der Randbereich kann damit als derjenige Bereich definiert sein, an dem das Abstrahlelement festlegbar ist und/oder an dem das Abstrahlelement, wenn das Arretierungsmittel in der Arretierstellung ist, festgelegt ist.

Alternativ oder ergänzend kann vorgesehen sein, dass das Infrarotheizmodul einen Berührschutz des Abstrahlelements aufweist, wodurch ein unbeabsichtigtes Berühren des Abstrahlelements verhinderbar ist.

Der Berührschutz kann verhindern, dass ein Anwender mit dem Abstrahlelement unbeabsichtigterweise in Kontakt kommt. Da das Abstrahlelement während des Einsatzes des Infrarotheizmoduls warm oder sogar heiß werden kann, schützt der Berührschutz vor Verbrennungen und erhöht damit die Sicherheit.

Beispielsweise ist der Berührschutz großmaschig ausgestaltet und/oder weist eine Größe, insbesondere eine Öffnungsgröße, von 120 mm × 40 mm auf.

Der Berührschutz weist vorzugsweise eine Wärmeleitfähigkeit von weniger als 1 W/mK, vorzugsweise von weniger als 0,8 W/mK, vorzugsweise von weniger als 0,5 W/mK, vorzugsweise von weniger als 0,4 W/mK, vorzugsweise von weniger als 0,25 W/mK, vorzugsweise von weniger als 0,1 W/mK, auf. Dadurch kann besonders zuverlässig sichergestellt sein, dass sich der Berührschutz nicht oder nur wenig durch die Infrarotstrahlung aufwärmt und dadurch auch wenig Wärme an das berührende Körperteil abgibt.

Alternativ oder ergänzend kann vorgesehen sein, dass der Berührschutz einstückig mit zumindest einem Teil des Arretierungsmittels und/oder des Gehäuses ausgebildet ist.

Indem der Berührschutz entsprechend ausgebildet ist, ist der Berührschutz immer vorgesehen, wenn das Infrarotheizmodul im Betrieb ist. Damit ist ein besonders sicherer Betrieb des Infrarotheizmoduls möglich.

Die Aufgabe wird durch die Erfindung gemäß einem **dritten Aspekt** dadurch gelöst, dass eine Vorrichtung gemäß dem ersten Aspekt der Erfindung, wobei zumindest eines der Infrarotheizmodule der Vorrichtung ein Infrarotheizmodul gemäß dem zweiten Aspekt der Erfindung aufweist oder darstellt, vorgeschlagen wird.

Der Erfindung liegt damit die überraschende Erkenntnis zugrunde, dass der Hautpartie die Wärmestrahlung besonders kontrolliert bereitgestellt werden kann, indem die Abstrahlcharakteristik der einzelnen Infrarotheizmodule berücksichtigt wird. Dadurch kann sichergestellt werden, dass eine lokale Überhitzung der Hautpartie in Folge eines zu hohen Wärmebeitrags mehrerer Infrarotheizmodule zuverlässig vermieden werden kann. Gleichermaßen können aber auch Bereiche mit zu geringer Wärmezufuhr vermieden werden, wodurch eine zuverlässigere Wärmeanwendung möglich ist.

Die Erfinder haben insoweit erkannt, dass die unterschiedlichen Abstrahlcharakteristiken besonders einfach aber dennoch wirkungsvoll dadurch berücksichtigt werden können, indem die Infrarotheizmodule mit unterschiedlicher Temperatur betrieben werden. Dadurch ist es überraschend einfach möglich, innerhalb des betrachteten Bereichs eine homogene Intensität der Wärmestrahlung, die sich aus überlagerten Beiträgen auch mehrerer Infrarotheizmodule ergeben kann, bereitzustellen.

Zur bevorzugten Definition der homogenen Intensität kann auf die Ausführungen im Zusammenhang mit dem ersten Aspekt der Erfindung verwiesen werden, die hier ganz entsprechend gelten.

Das homogene Wärmefeld (also eine homogene Intensität der Wärmestrahlung) ermöglicht dabei nicht nur eine, vor allem hinsichtlich der zulässigen Grenzwerte, besonders kontrollierte Wärmebehandlung. Zusätzlich erlaubt ein Bereich mit einem homogenen Wärmefeld auch eine Toleranz gegenüber einer relativen Bewegung von Vorrichtung und Hautpartie. Denn auch bei einer relativen Verschiebung ist eine gute Wärmebehandlung durchführbar.

Indem außerdem auch ein austauschbares Abstrahlelement in wenigstens einem der Infrarotheizmodule vorgesehen ist, kann die Abstrahlcharakteristik zumindest des einen Infrarotheizmoduls individuell angepasst werden.

Durch das Zusammenspiel des ersten und des zweiten Erfindungsaspekts wurde durch die Erfinder überraschend eine Möglichkeit gefunden, die von der Vorrichtung bereitgestellte Wärmestrahlung, welcher beispielsweise dann die Hautpartie aussetzbar ist, noch besser definieren zu können, und damit ein noch zuverlässiger eine homogene Intensität der Wärmestrahlung bereitgestellt werden kann.

Mit anderen Worten, durch die individuelle Temperatureinstellung der Infrarotheizmodule einerseits und den Einsatz des Abstrahlelements andererseits lassen sich nach Erkenntnissen der Erfinder Synergieeffekte aus beiden Maßnahmen erzielen, die zu einer Wärmestrahlung mit besonders vorteilhaften, da homogenen, Intensitätsverteilung, führen.

### Kurzbeschreibung der Figuren

Weitere Merkmale und Vorteile der Erfindung ergeben sich aus der nachfolgenden Beschreibung, in der bevorzugte Ausführungsformen der Erfindung anhand schematischer Zeichnungen erläutert werden.

Dabei zeigen:

Fig. 1a    eine schematische Querschnittsansicht einer erfindungsgemäßen Vorrichtung in einer ersten Konfiguration;

Fig. 1b    die Ansicht aus Fig. 1a mit eingezeichnetem Betrachtungsbereich;

Fig. 2     eine schematische Frontalansicht der Vorrichtung aus Fig. 1a;

Fig. 3     eine schematische Querschnittsansicht der erfindungsgemäßen Vorrichtung aus Fig. 1a in einer zweiten Konfiguration;

Fig. 4     eine schematische Querschnittsansicht eines erfindungsgemäßen Infrarotheizmoduls in einer ersten Ausführungsform;

Fig. 5     eine schematische Frontalansicht des Infrarotheizmoduls aus Fig. 4;

Fig. 6a    eine schematische Seitenansicht eines erfindungsgemäßen Infrarotheizmoduls in ei-

Fig. 6b    eine vergrößerte Darstellung eines Ausschnitts aus Fig. 6a;

Fig. 7    eine schematische Frontalansicht des Infrarotheizmoduls aus Fig. 6;

Fig. 8    einen Schnellspannrahmen;

Fig. 9    eine schematische Frontalansicht einer erfindungsgemäßen Vorrichtung mit einem erfindungsgemäßen Infrarotheizmodul;

Fig. 10a    eine noch weitere Ausführungsform einer erfindungsgemäßen Vorrichtung in einer perspektivischen Ansicht von schräg vorne;

Fig. 10b    die Vorrichtung aus Fig. 10a in einer perspektivischen Ansicht von schräg hinten;

Fig. 10c    einen Ausschnitt mit einem oberen Teil der Vorrichtung aus Fig. 10a in einer kompakten Konfiguration in einer perspektivischen Ansicht von schräg hinten;

Fig. 10d    die Vorrichtung aus Fig. 10c mit hervorgehobenen den Infrarotheizmodulen in einer perspektivischen Ansicht von vorne;

Fig. 11    ein Abstützelement in einer vergrößerten Darstellung; und

Fig. 12    eine alternative Ausführung eines Podestelements.

Beispiele

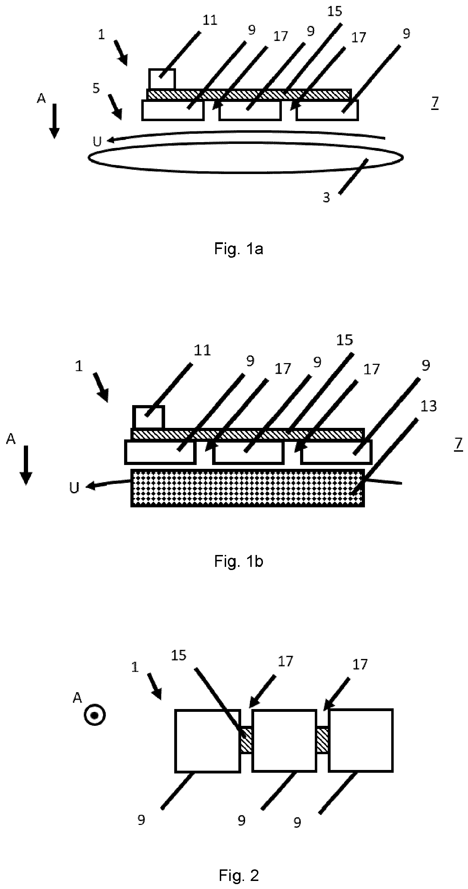

Figur 1 zeigt eine schematische Querschnittsansicht einer erfindungsgemäßen Vorrichtung 1 in einer ersten Konfiguration.

Die Vorrichtung 1 ist in Fig. 1 bei einem Einsatz gezeigt, bei dem sie relativ zu und an einem Körperteil, hier einem Rumpf 3, eines Menschen angeordnet ist. Der Rumpf 3 weist eine Hautpartie 5 des Rückens des Menschen auf. Die Zeichenebene der Figur 1 und damit die Schnittebene der Vorrichtung 1 entspricht einer spezifischen Transversalebene 7 des Menschen.

Die Vorrichtung 1 weist insgesamt drei Infrarotheizmodule 9 auf. Alle der Infrarotheizmodule 9 weisen eine Schnittfläche mit der spezifischen Transversalebene 7 auf. Mit anderen Worten ausgedrückt, sind also die drei Infrarotheizmodule 3 entlang einer Umfangsrichtung U an dem Rumpf 3, genauer gesagt, dem Rücken des Menschen, angeordnet.

Jedes der Infrarotheizmodule 9 ist auf einer individuellen Temperatur betreibbar. Diese Betriebstemperatur bestimmt die von dem jeweiligen Infrarotheizmodul abgegebene Wärmestrahlung. Vorliegend ist hauptsächlich die in Richtung der Hautpartie 5 abgegebene Wärmestrahlung relevant.

Die Vorrichtung 1 weist auch ein Kontrollmodul 11 auf. Dieses ist dazu eingerichtet, die Betriebstemperaturen der einzelnen Infrarotheizmodule 9 zu kontrollieren. Dies erfolgt dabei derart, dass die beiden seitlichen Infrarotheizmodule (also die in Fig. 1 links und rechts befindlichen Infrarotheizmodule 9) auf einer gleichen Betriebstemperatur betrieben werden, also entsprechend kontrolliert werden. Demgegenüber wird das mittlere der drei Infrarotheizmodule 9 in Fig. 1 auf einer niedrigeren Temperatur betrieben, also von dem dazu eingerichteten Kontrollmodul 11 entsprechend kontrolliert.

Vorliegend beträgt die Betriebstemperatur der beiden seitlichen Infrarotheizmodule 9 250 °C und die Betriebstemperatur des mittleren Infrarotheizmoduls beträgt 200 °C.

Indem also die beiden seitlichen Infrarotheizmodule 9 auf gleicher Temperatur betrieben werden, und das mittlere Infrarotheizmodul 9 auf einer niedrigeren Betriebstemperatur betrieben wird, ist innerhalb eines betrachteten Bereichs 13 (vgl. Fig. 1b) entlang einer Abstandsrichtung A zwischen Vorrichtung 1 und Rumpf 3 und der Umfangsrichtung U die beim Einsatz der Vorrichtung 1 von der Vorrichtung 1 bereitgestellte Intensität der Wärmestrahlung zum Zuführen an die Hautpartie 5 homogen.

In einer Ausführungsform weist die Vorrichtung 1 außerdem ein (in Fig. 1a nicht gezeigtes) Abstützelement zum zumindest teilweisen Abstützen der Vorrichtung 1 an dem Menschen auf.

Figur 1b zeigt dabei identisch die Vorrichtung 1 der Fig. 1a, wobei zusätzlich der betrachtete Bereich 13 als Überlagerung illustriert ist. Der betrachtete Bereich 13 erstreckt sich seitlich entlang des mittleren und jeweils Teilen der beiden seitlichen Infrarotheizmodule 9. Der betrachtete Bereich 13 liegt (ganz oder teilweise) auch zwischen der Vorrichtung 1 und dem Rumpf 3.

Das heißt, innerhalb des betrachteten Bereichs 13 ist entlang der Umfangsrichtung U und der Abstandsrichtung A die Schwankung der Intensität der Wärmestrahlung begrenzt. Ausgehend von jedem Punkt innerhalb des Bereichs 13 kann mithin in Abstandsrichtung A und/oder in Umfangsrichtung U die homogene Wärmestrahlung festgestellt werden. Das heißt für die Vorrichtung 1, dass die maximale und die minimale Intensität der Wärmestrahlung, $I_{max}$ und $I_{min}$, im Bereich 13 der Ungleichung

$$2\frac{I_{max}-I_{min}}{I_{max}+I_{min}} \leq 1,9$$

genügen.

Das ermöglicht vorliegend eine zuverlässige Wärmebehandlung des Rumpfes 3 und dort insbesondere der Hautpartie 5. Denn trotz der Krümmung des Rückens und damit trotz des gekrümmten Verlaufs der Hauptpartie 5, kann in dem Bereich 13 eine homogene Wärmezufuhr zu der Hautpartie 5 ermöglicht werden. Außerdem ist die Vorrichtung 1 dadurch unempfindlich gegenüber relativen Verschiebungen zwischen Rumpf 3 und Vorrichtung 1.

Wie der Fig. 1 entnommen werden kann, sind Abstandsrichtung A und Umfangsrichtung U beide parallel zur Transversalebene 7. Der Bereich 13 (Fig. 1b) könnte sich außerdem auch teilweise entlang der Richtung senkrecht zur Zeichenebene erstrecken, was jedoch vorliegend nicht näher betrachtet wird. Im gesamten Volumenbereich (von dem der Bereich 13 die Schnittfläche mit der spezifischen Transversalebene 7 / Zeichenebene darstellt) könnte dann entlang der beiden Richtungen A und U die homogene Intensität der Wärmestrahlung festgestellt werden.

Die einzelnen Infrarotheizmodule 9 sind relativ zueinander entlang eines von der Vorrichtung aufgewiesenen Führungselements 15 verschiebbar und sind damit relativ zueinander veränderlich positionierbar.

Die einzelnen Infrarotheizmodule 9 sind durch Luftspalte 17 thermisch voneinander entkoppelt. Außerdem sind die einzelnen Infrarotheizmodule 9 durch eine (in Fig. 1a nicht dargestellte) Isolierung thermisch von dem Führungselement 15 entkoppelt.

Fig. 2 zeigt eine schematische Frontalansicht der Vorrichtung 1 aus Fig. 1a, entgegen der Richtung A.

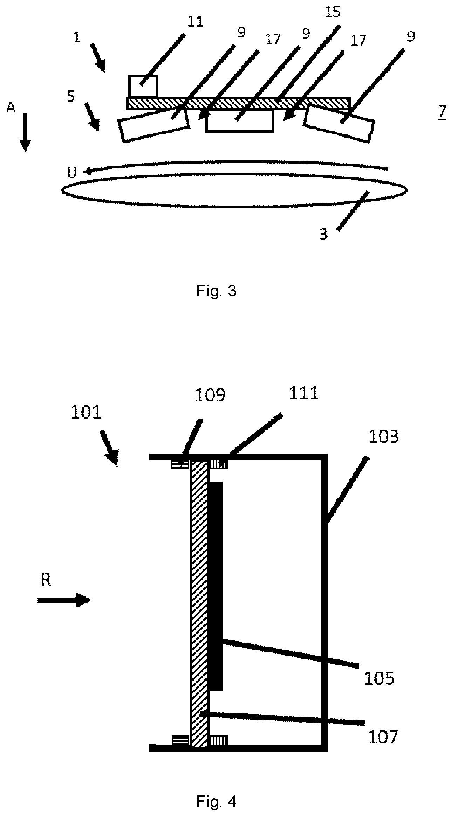

Fig. 3 zeigt eine schematische Querschnittsansicht der erfindungsgemäßen Vorrichtung 1 aus Fig. 1a in einer zweiten Konfiguration.

In der zweiten Konfiguration sind die beiden äußeren Infrarotheizmodule 9 gegenüber dem mittleren Infrarotheizmodul 9 abgewinkelt. Dadurch lässt sich die Form des Rumpfes 3 und damit die Krümmung der Hauptpartie 5 besser nachbilden und die Wärmestrahlung besser der Hautpartie 5 zuführen. Der Winkel zwischen der Normalen eines seitlichen und des mittleren Infrarotheizmoduls 9 liegt innerhalb der spezifischen Ebene 7.

Fig. 4 zeigt eine schematische Querschnittsansicht eines erfindungsgemäßen Infrarotheizmoduls 101 in einer ersten Ausführungsform.

Das Infrarotheizmodul 101 weist ein Gehäuse 103 auf und ein innerhalb des Gehäuses angeordnetes Infrarotstrahlerelement 105 in Form eines Heizdrahtes. Außerdem weist das Infrarotheizmodul 101 ein scheibenförmiges, rechteckiges Abstrahlelement 107 auf, das an dem Gehäuse 103 lösbar angeordnet ist.

Genauer gesprochen ist das Abstrahlelement 107 von einem Arretierungsmittel 109 lösbar an einem Rand 111 des Gehäuses 103 gehalten, indem das Arretierungsmittel 109 in der in Fig. 4 gezeigten Arretierstellung rastend in das Gehäuse 103 eingreift und die auf dem Abstrahlelement 107 aufliegende Auflagefläche des Arretierungsmittels 109 das Abstrahlelement 107 an das Gehäuse 103, nämlich an dessen Rand 111, und an das Infrarotstrahlerelement 105 drückt. Dadurch wird das Abstrahlelement 107 form- und kraftschlüssig an dem Gehäuse 103 gehalten. Außerdem steht es auch in direktem Kontakt mit dem Infrarotstrahlerelement 105.

Indem das Arretierungsmittel 109 von dem Gehäuse 103 gelöst wird, das Arretierungsmittel 109 also in eine (in der Fig. 4 nicht dargestellten) Entnahmestellung überführt wird, lässt sich auch das Abstrahlelement 107 aus dem Gehäuse 103 entnehmen und zum Beispiel gegen ein anderes Abstrahlelement austauschen.

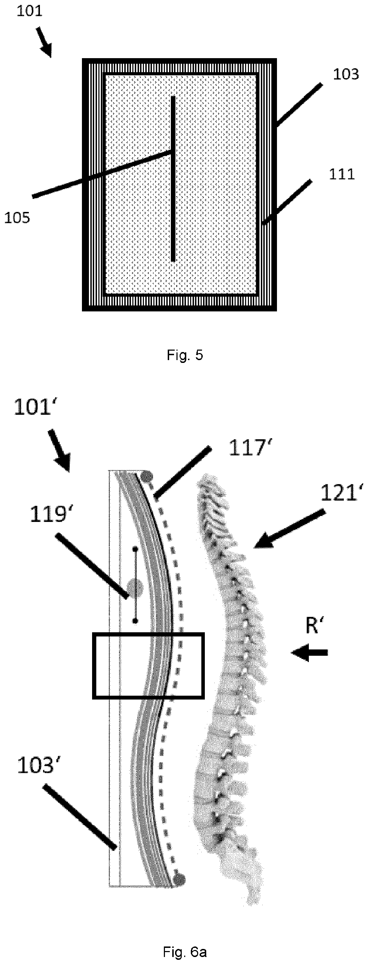

Fig. 5 zeigt eine schematische Frontalansicht des Infrarotheizmoduls 101 aus Fig. 4, und zwar entlang der Blickrichtung R in Fig. 4. Allerdings ist in Fig. 5 das Arretierungsmittel 109 und das Abstrahlelement 107 entfernt, so dass der Blick in das Innere des Gehäuses 103 frei ist. Gut zu erkennen ist dadurch der Rand 111 des Gehäuses 103, auf dem das Abstrahlelement 107 zum Aufliegen kommt. Nicht dargestellt ist in Fig. 5 eine Befestigung des Infrarotstrahlerelements 105 innerhalb des Gehäuses 103.

Mit dem Infrarotheizmodul 101 kann einer Hautpartie, die sich vor dem Abstrahlelement 107 befindet, Wärme zugeführt werden. Dazu wird beim Einsatz des Infrarotheizmoduls 101 der Heizdraht 105 mit Spannung beaufschlagt, wodurch dieser durch Konduktion das Abstrahlelement 107 aufwärmt. Dieses gibt dann seinerseits Wärmestrahlung ab.

Das Abstrahlelement 107 ermöglicht es dadurch, die Wärmestrahlung zu beeinflussen. Beispielsweise kann die Wärme über einen eher flächigen Bereich abgestrahlt werden (im Gegensatz zu dem eher linienförmigen Heizdraht 105). Das Abstrahlen erfolgt dann beispielsweise in Richtung der vor dem Infrarotheizmodul 101 befindlichen Hautpartie, also insbesondere entgegen der Richtung R.

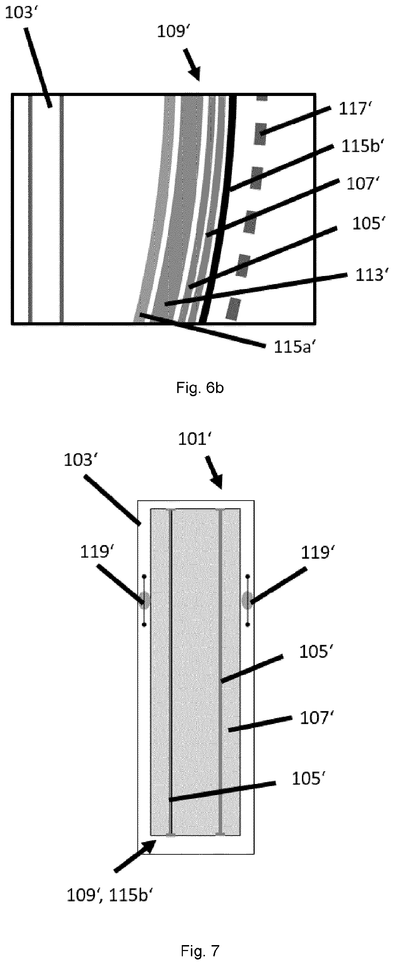

Fig. 6a zeigt eine schematische Seitenansicht eines erfindungsgemäßen Infrarotheizmoduls 101' in einer zweiten Ausführungsform. Fig. 6b zeigt den in Fig. 6a durch einen Kasten markierten Ausschnitt in einer vergrößerten Darstellung.

Merkmale des Infrarotheizmoduls 101', die funktional oder konstruktiv ähnlich zu denen des in Bezug auf die Figs. 4 und 5 besprochenen Infrarotheizmoduls 101 sind, sind mit gleichen, jedoch einfach gestrichenen Bezugszeichen versehen. Es kann daher in Bezug auf diese Merkmale ergänzend auch auf die oben in Bezug auf Figuren 4 und 5 gemachten Ausführungen verwiesen werden, sofern sich aus dem Zusammenhang nichts anderes ergibt.

Das Infrarotheizmodul 101' weist ein Gehäuse 103' auf, innerhalb dessen die einzelnen Komponenten des Infrarotheizmodul 101' angeordnet sind. Insbesondere also das Abstrahlelement 107' mit dahinter angeordneten Infrarotstrahlerelementen 105' sowie einer dahinter angeordneten thermischen Isolierung 113' in Form einer, beispielsweise 10 mm dicken, Aereogelschicht (alles entlang einer Blickrichtung R' von rechts in den Figuren 6a und 6b betrachtet). Insbesondere ist also das Infrarotstrahlerelementen 105' direkt an dem Abstrahlelement 107' angeordnet, so dass Wärme von dem Infrarotstrahlerelementen 105' an das Abstrahlelement 107' per Konduktion übertragen werden kann. Es wäre auch grundsätzlich eine beabstandete Anordnung von Infrarotstrahlerelementen 105' und Abstrahlelement 107' denkbar, wobei dann auch oder ausschließlich die von dem Infrarotstrahlerelementen 105' abgegebene Wärmestrahlung zu einer Aufwärmung des Abstrahlelements 107' führen würde.

Abstrahlelement 107', Infrarotstrahlerelement 105' und Isolierung 113' sind sandwichartig zwischen einem hinteren Teil 115a' und einem vorderen Teil 115b' eines Arretierungsmittels 109' in Form eines Schnellspannrahmens innerhalb des Gehäuses 103' vorgesehen.

Durch den Spannrahmen 109' lässt sich vor allem das Abstrahlelement 107' leicht auswechseln und so die Abstrahlcharakteristik des Infrarotheizmoduls 101' besonders einfach anpassen.

Außerdem weist das Infrarotheizmodul 101' einen Berührungsschutz 117' auf, der verhindert, dass das Abstrahlelement 107' versehentlich berührt wird (insbesondere dort, wo kein Spannrahmen 115b' sozusagen "im Weg" ist). Ferner weist das Infrarotheizmodul 101' auch Temperatursensoren 119' auf. Die Temperatursensoren 119' sind vertikal in der Höhe verschiebbar.

In Figur 6a ist außerdem der S-förmig gekrümmte Verlauf einer Wirbelsäule 121' eines Menschen dargestellt. Wie sich schön zeigt, sind die Komponenten 117', 115b', 107', 105', 113' und 115a' derart geformt, dass sie den Verlauf der Wirbelsäule 121' annähernd nachbilden. Dadurch ist das Infrarotheizmodul 101' sehr ergonomisch, passt sich dem Verlauf des Rückens eines Menschen an und ermöglicht dadurch eine zuverlässige und effektive Wärmebehandlung einer Hautpartie des Rückens.

Fig. 7 zeigt eine schematische Frontalansicht des Infrarotheizmoduls 101' aus Fig. 6a, und zwar entlang der Blickrichtung R' in Fig. 6a.

Anhand Fig. 7 ist erkennbar, dass das Infrarotheizmodul 101' zwei Temperatursensoren 119' aufweist (in Form von Infrarot-Temperatursensoren). Die beiden Infrarotstrahlerelemente 105' sind als linienförmige Heizdrähte ausgestaltet. Da sich diese (aus der Blickrichtung von vorne) hinter dem Abstrahlelement 107' befinden, dürften die beiden in Fig. 7 eigentlich nicht sichtbar sein. Jedoch sind sie zur Illustration dennoch in Fig. 7 dargestellt. Der Übersichtlichkeit halber nicht dargestellt ist in Fig. 7 hingegen der Berührungsschutz 117'.

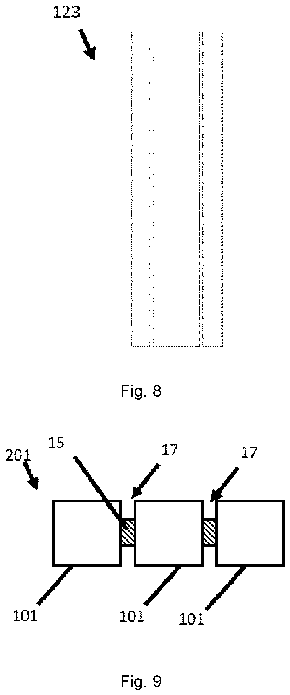

Fig. 8 zeigt einen Schnellspannrahmen 123, wie er beispielsweise in dem Infrarotheizmodul 101, das in Bezug auf Figuren 4 und 5 besprochen wurde oder in dem Infrarotheizmodul 101', das in Bezug auf Figuren 6 und 7 besprochen wurde, eingesetzt werden kann.

Der Schnellspannrahmen 123 ist aus Blech.

Fig. 9 zeigt eine schematische Frontalansicht einer erfindungsgemäßen Vorrichtung 201 mit drei erfindungsgemäßen Infrarotheizmodulen 101.

Die Vorrichtung 201 ist dabei vorliegend wie die Vorrichtung 1 aufgebaut, wobei die Infrarotheizmodule 9 der Vorrichtung 1 durch Infrarotheizmodule 101 realisiert sind. Daher kann hinsichtlich der weiteren Beschreibung der Vorrichtung 201 auf die oben gemachten Ausführungen zur Vorrichtung 1 und zum Infrarotheizmodul 101 verwiesen werden.

In einer Ausführungsform weist die Vorrichtung 201 außerdem ein (in Fig. 9 nicht gezeigtes) Abstützelement zum zumindest teilweisen Abstützen der Vorrichtung 201 an dem Menschen auf.

Die Vorrichtung 201 ermöglicht damit eine homogene Intensität der Wärmestrahlung, indem die einzelnen Infrarotheizmodule 101 mit individuellen Betriebstemperaturen betrieben werden, wobei die Homogenität durch das austauschbare Abstrahlelement der Infrarotheizmodule 101 besonders auf das jeweilige Anwendungsszenario abgestimmt werden kann.

Eine weitere Ausführungsform einer erfindungsgemäßen Vorrichtung ist ganz ähnlich zur in Fig. 9 dargestellten Vorrichtung 201, mit jedoch dem Unterschied, dass die Infrarotheizmodule durch Infrarotheizmodule 101' realisiert sind.

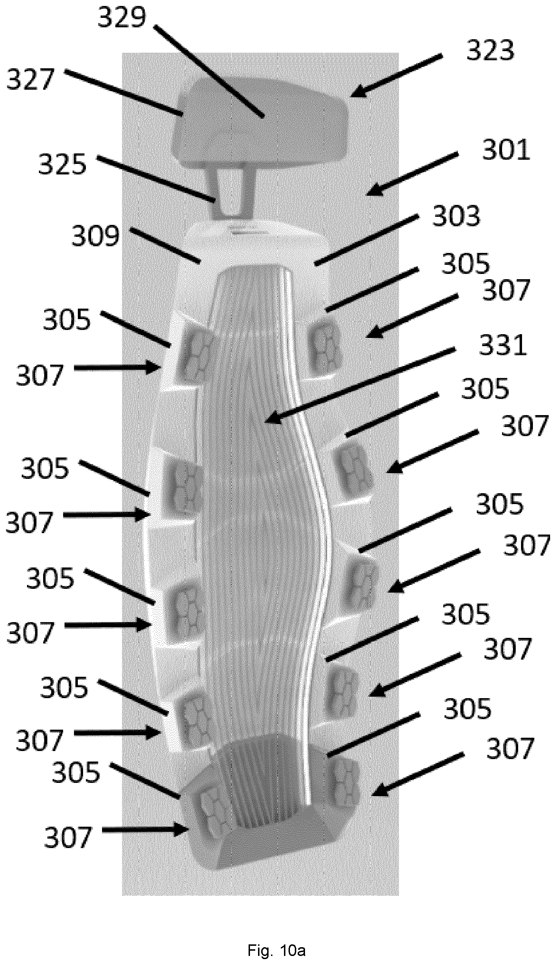

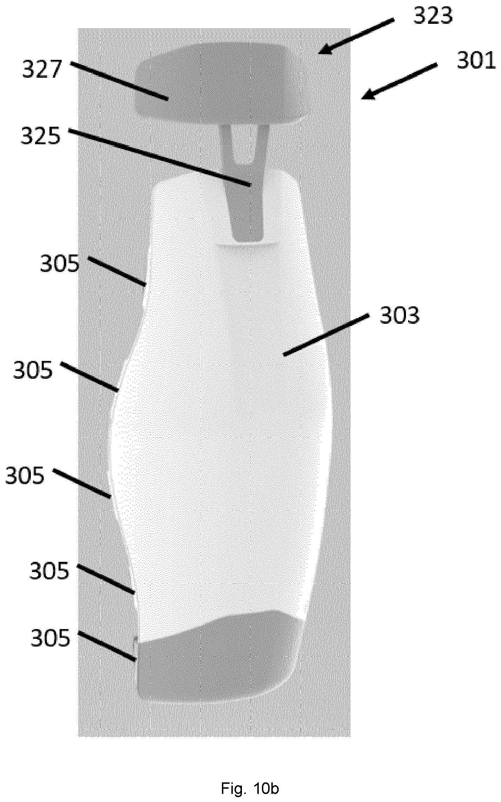

Fig. 10a zeigt eine noch weitere Ausführungsform einer erfindungsgemäßen Vorrichtung 301 in einer perspektivischen Ansicht von schräg vorne. Fig. 10b zeigt die Vorrichtung 301 in einer perspektivischen Ansicht von schräg hinten.

Die Vorrichtung 301 weist ein Gehäuse 303 auf, innerhalb dessen drei (in Figs. 10a und 10b nicht dargestellte) Infrarotheizmodule angeordnet sind.

Die Vorrichtung 301 weist an der Frontpartie des Gehäuses 303 zehn pyramidenstumpfförmige Podestelemente 305 auf. Diese bilden eine Erhöhung aus.

Die Vorrichtung 301 weist außerdem zehn gleichartige Abstützelemente 307 auf. Diese bilden eine Rückenlehne aus und dienen daher dazu, die Vorrichtung 301 an einem Lebewesen, an dessen Hautpartie mit der Vorrichtung 301 Wärme zugeführt werden soll, abstützen zu können. Jedes Abstützelement ist auf jeweils einem der Podestelemente 305 und damit auch indirekt an der Frontpartie 309 des Gehäuses 303 angeordnet.

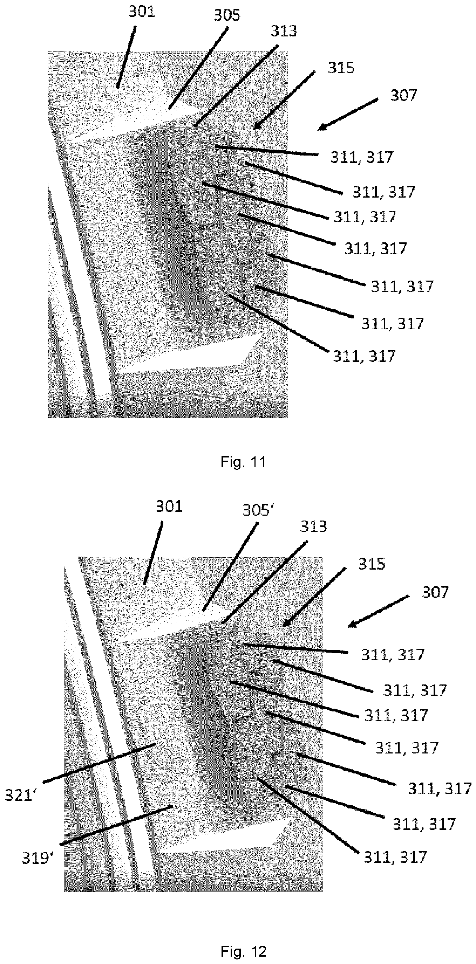

Fig. 11 zeigt ein Abstützelement 307 in vergrößerter Darstellung. Da alle Abstützelemente 307 in dieser Ausführungsform gleich sind, genügt es, ein einziges davon exemplarisch anhand Fig. 11 näher zu erläutern.

Das Abstützelement 307 (siehe Fig. 11) weist jeweils mehrere Einzelelemente 311 auf, die stoffschlüssig auf einem von dem Abstützelement 307 aufgewiesenen elastischen Grundelement 313 aus Silikon nebeneinander angeordnet sind. Werden die starren Einzelelemente 311 durch das Lebewesen belastet, sind diese gegeneinander bewegbar, da die Einzelelemente 311 in die Silikonschicht des elastischen Grundelements 313 gedrückt werden können und dadurch jeweils ihre Position und Lage individuell verändert werden kann.

Dadurch ist der Verlauf der von dem Abstützelement 307 bereitgestellten spezifischen Oberfläche 315 an den Verlauf einer Oberfläche des Lebewesens (wie die eines Rückenbereichs dessen) anpassbar. Jedes Einzelelement 311 weist dabei einen Oberflächenbereich 317 auf, der einen Teil der spezifischen Oberfläche 315 des Abstützelements 307 bereitstellt. Fünf der sieben Einzelelemente 311 des Abstützelements 305 stellen insofern eine wabenförmige, also sechseckige, Oberfläche bereit. Die anderen beiden jeweils eine viereckige Oberfläche.

In Fig. 12 ist eine alternative Ausführung zu den zuvor beschriebenen Podestelementen 305 mit darauf angeordnetem Abstützelement 307 dargestellt. Innerhalb des Podestelements 305' ist ein Infrarot-Temperatursensor vorgesehen, der dazu eingerichtet ist eine lokale Hauttemperatur des Lebewesens zu messen. Die Seitenfläche 319' des Podestelements 305' ist in einem Bereich 321' für die Infrarot-Temperaturmessung durchlässig. Indem die Seitenfläche 319' leicht schräg verläuft, hat der Infrarot-Temperatursensor einen guten Blickwinkel auf die Hautpartie.

Wieder in Bezug auf Fig. 10a und Fig. 10b. Die Vorrichtung 301 weist ferner eine Kopfstütze 323 auf, die mit einem Haltemittel 325 beabstandet von und an dem Gehäuse 303 befestigt ist. Die Kopfstütze 323 weist dabei eine Plastikschale 327 und ein daran angeordnetes flexibles Netzgewebe 329 auf, das eine Öffnung der Plastikschale 327 überspannt.

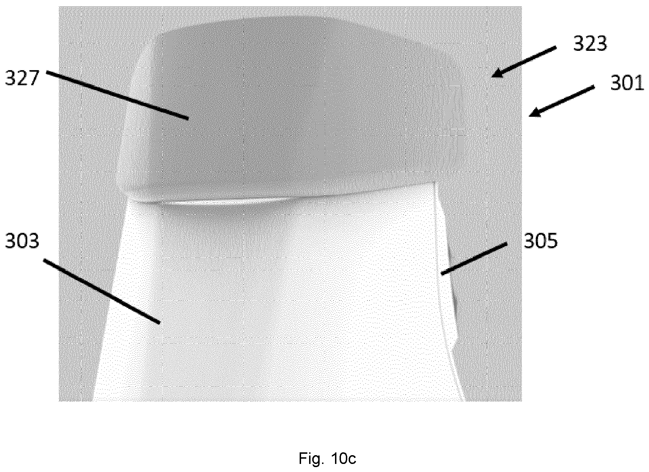

Fig. 10c zeigt einen Ausschnitt mit einem oberen Teil der Vorrichtung 301 aus Fig. 10a in einer kompakten Konfiguration in einer perspektivischen Ansicht von schräg hinten. In dieser kompakten Konfiguration (Fig. 10c) ist die Kopfstütze 323 als Gehäuseabdeckung im oberen Bereich der Vorrichtung 301 an dem Gehäuse 305 lösbar befestigt, etwa da die Vorrichtung nicht im Einsatz ist.

Die Vorrichtung 301 weist einen gitterförmigen Berührschutz 331 auf, der ein unbeabsichtigtes Berühren der Infrarotheizmodule verhindert.

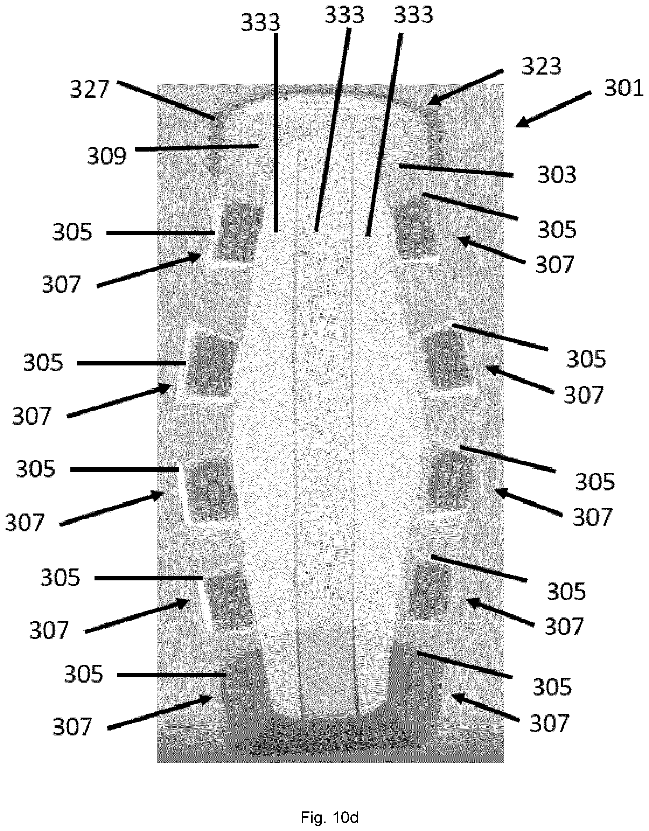

Fig. 10d zeigt die Vorrichtung 301 in der kompakten Konfiguration aus Fig. 10c ohne den Berührschutz aber mit den drei Infrarotheizmodulen 333. Diese erstrecken sich beim Einsatz der Vorrichtung 301 streifenförmig entlang einer zu einer Transversalebene des Lebewesens senkrecht verlaufenden Richtung.

Die in der vorangehenden Beschreibung, in den Ansprüchen und in den Zeichnungen offenbarten Merkmale können sowohl einzeln als auch in beliebiger Kombination wesentlich für die Erfindung in ihren verschiedenen Ausführungsformen sein.

Bezugszeichenliste

| | |
|---|---|
| 1,201 | Vorrichtung |
| 3 | Rumpf |
| 5 | Hautpartie |
| 7 | Transversalebene |
| 9 | Infrarotheizmodul |
| 11 | Kontrollmodul |
| 13 | Bereich |
| 15 | Führungselement |
| 17 | Luftspalt |
| 101, 101' | Infrarotheizmodul |
| 103, 103' | Gehäuse |
| 105, 105' | Infrarotstrahlerelement |
| 107, 107' | Abstrahlelement |
| 109, 109' | Arretierungsmittel |
| 111 | Rand |
| 113' | Thermische Isolierung |
| 115a', 115b' | Teile eines Spannrahmens |
| 117' | Berührungsschutz |
| 119' | Temperatursensor |
| 121' | Wirbelsäule |
| 123 | Schnellspannrahmen |
| 301 | Vorrichtung |
| 303 | Gehäuse |
| 305, 305' | Podestelement |
| 307 | Abstützelement |
| 309 | Frontpartie |
| 311 | Einzelelement |
| 313 | Elastisches Grundelement |
| 315 | Spezifischen Oberfläche |
| 317 | Oberfläche |
| 319' | Seitenfläche |
| 321' | Bereich |
| 323 | Kopfstütze |
| 325 | Haltemittel |
| 327 | Plastikschale |
| 329 | Netzgewebe |
| 331 | Berührschutz |
| 333 | Infrarotheizmodul |
| A | Abstandsrichtung |
| R, R' | Richtung |
| U | Umfangsrichtung |

**Patentansprüche**

1. Vorrichtung zum Zuführen von Wärme an eine Hautpartie eines Lebewesens, wobei die Vorrichtung drei oder mehr als drei Infrarotheizmodule aufweist, und wobei die Vorrichtung zumindest ein Abstützelement zum zumindest teilweisen Abstützen der Vorrichtung an dem Lebewesen aufweist.

2. Vorrichtung nach Anspruch 1, wobei die Vorrichtung wenigstens ein Gehäuse aufweist, und wobei vorzugsweise innerhalb eines von dem Gehäuse zumindest bereichsweise eingeschlossenen Volumens die Infrarotheizmodule angeordnet sind.

3. Vorrichtung nach einem der vorangehenden Ansprüche, wobei (i) das zumindest eine Abstützelement an einer Seitenpartie und/oder an einer Frontpartie des Gehäuses direkt oder indirekt angeordnet ist, (ii) mehrere gleichartige Abstützelemente an der Vorrichtung, insbesondere ihrer Seiten- und/oder Frontpartie, angeordnet sind und/oder (iii) das zumindest eine Abstützelement zum zumindest teilweisen Abstützen der Vorrichtung an einem Rückenbereich des Lebewesens geeignet ist.

4. Vorrichtung nach einem der vorangehenden Ansprüche, wobei das Abstützelement eine spezifische Oberfläche bereitstellt, die vorzugsweise zum Abstützen der Vorrichtung an dem Lebewesen mit einer Kontaktoberfläche, insbesondere eines Rückenbereichs, des Lebewesens in Kontakt bringbar ist, wobei der Verlauf und/oder die Gestalt der spezifischen Oberfläche zumindest abschnittsweise und/oder bereichsweise an den Verlauf und/oder die Gestalt der Kontaktoberfläche anpassbar ist.

5. Vorrichtung nach Anspruch 4, wobei das Abstützelement mit spezifischer Oberfläche mehrere, vorzugsweise gegeneinander bewegbare, insbesondere in ihrer Position und/oder Lage veränderbare, und/oder, insbesondere durch Komprimierung, in ihrer Form und/oder Gestalt reversibel veränderbare, Einzelelemente aufweist und jedes Einzelelement mit einem Oberflächenbereich einen Teil der spezifischen Oberfläche des Abstützelements bereitstellt.

6. Vorrichtung nach Anspruch 5, wobei das Abstützelement mit spezifischer Oberfläche ein elastisches Grundelement, insbesondere bestehend aus oder aufweisend Silikon, aufweist, auf dem die Einzelelemente angeordnet sind, und insbesondere stoffschlüssig mit dem elastischen Grundelement verbunden sind, wobei vorzugsweise (i) die Einzelelemente, vorzugsweise nebeneinander und/oder flächig, auf dem elastischen Grundelement angeordnet sind, (ii) das elastische Grundelement schichtförmig ausgebildet ist, (iii) das elastische Grundelement, insbesondere unmittelbar, zwischen einerseits den Einzelelementen und andererseits dem Gehäuse angeordnet ist, vorzugsweise das elastische Grundelement direkt auf dem Gehäuse angeordnet ist, (iv) das elastische Grundelement quaderförmig ist und/oder (v) das elastische Grundelement eine Dicke von wenigstens 0,5 cm und/oder von maximal 10 cm aufweist.

7. Vorrichtung nach einem der Ansprüche 5 bis 6, wobei (i) die Einzelelemente selbst nichtkompressibel sind und/oder als Material Kunststoffe, Kunstleder, Leder und/oder sonstige Naturmaterialien aufweisen oder daraus bestehen und/oder (ii) das Abstützelement mit spezifischer Oberfläche 2 oder mehr als 2, vorzugsweise 5 oder mehr als 5, vorzugsweise 10 oder mehr als 10, und/oder 20 oder weniger als 20, vorzugsweise 15 oder weniger als 15, vorzugsweise 10 oder weniger als 10, vorzugsweise 7 oder weniger als 7, vorzugsweise 5 oder weniger als 5, vorzugsweise 3 oder weniger als 3, Einzelelemente aufweist.

8. Vorrichtung nach einem der vorangehenden Ansprüche, wobei die Vorrichtung, insbesondere an der Seitenpartie und/oder an der Frontpartie, zumindest ein, vorzugsweise pyramidenstumpfförmige oder parallelepipedförmige, Podestelement aufweist, vorzugsweise mehrere solcher Podestelemente aufweist, und wobei vorzugsweise die Podestelemente von dem Gehäuse ausgebildet werden.

9. Vorrichtung nach einem der vorangehenden Ansprüche, wobei die Vorrichtung zumindest einen Infrarot-Temperatursensor aufweist, der insbesondere dazu eingerichtet ist, eine lokale Hauttemperatur des Lebewesens zu messen, wobei vorzugsweise der Sensor (i) an dem Gehäuse angeordnet ist, (ii) derart an der Vorrichtung, insbesondere an dem Gehäuse, angeordnet ist, dass der Sensor beim Einsatz der Vorrichtung zwischen den Infrarotheizmodulen und der Hautpartie des Lebewesens angeordnet ist, (iii) außerhalb des von dem Gehäuse zumindest bereichsweise eingeschlossenen Volumens angeordnet ist, (iv) im Bereich eines Podestelements angeordnet ist, (v) an einem Podestelement angeordnet ist, insbesondere an einer Seitenfläche des Podestelements, und/oder (vi) ganz oder teilweise innerhalb eines Podestelements angeordnet ist, und wobei vorzugsweise das Podestelement, insbesondere einer seiner Seitenflächen, zumindest bereichsweise einen für die Infrarot-Temperaturmessung durchlässigen Wandbereiche aufweist.

10. Vorrichtung nach einem der Ansprüche 8 bis 9, wobei das Abstützelement mit spezifischer Oberfläche, vorzugsweise alle Abstützelemente mit spezifischer Oberfläche, jeweils auf einem Podestelement angeordnet ist und/oder auf Silikon gelagert angeordnet ist.

11. Vorrichtung nach einem der vorangehenden Ansprüche, wobei das Abstützelement mit spezifischer Oberfläche (i) eine Rückenlehne ausbildet und/oder (ii) zerstörungsfrei lösbar und/oder austauschbar an der Vorrichtung, insbesondere an dem Podestelement, angeordnet ist.

12. Vorrichtung nach einem der vorangehenden Ansprüche, wobei die Vorrichtung einen Berührschutz aufweist, wodurch ein unbeabsichtigtes Berühren der Infrarotheizmodule verhinderbar ist, wobei der Berührschutz vorzugsweise (i) in einer von dem Gehäuse ausgebildeten Öffnung angeordnet ist und/oder diese zumindest teilweise ausfüllt und/oder (ii) gitterförmig ist, insbesondere mehrere zueinander beabstandete Gitterstäbe und/oder Saiten aufweist oder daraus besteht.

13. Vorrichtung nach einem der vorangehenden Ansprüche, wobei die Infrarotheizmodule jeweils ein lasergeschnittenes Heizelement oder ein Flächenheizelement aus Kunstglimmer aufweisen und/oder eine Abdeckung aus, insbesondere, eloxiertem, Aluminium aufweisen; und/oder
wobei die Infrarotheizmodule jeweils zumindest ein Infrarotstrahlerelement in Form eines Heizdrahtes, einer Folienheizung, eines Heizgewebes, von Mikanit, einer Dickschichtheizung und/oder einer Silikonheizmatte, aufweist, vorzugsweise in Form eines Carbongewebes.

14. Vorrichtung nach einem der vorangehenden Ansprüche, wobei die Infrarotheizmodule beim Einsatz der Vorrichtung derart relativ zu und/oder an dem Lebewesen angeordnet sind, dass für eine definierte oder definierbare spezifische Transversalebene des Lebewesens jedes der Infrarotheizmodule eine Schnittfläche mit der spezifischen Transversalebene hat, wobei vorzugsweise jedes Infrarotheizmodul auf einer individuellen Betriebstemperatur betreibbar ist, und/oder

beim Einsatz der Vorrichtung die Betriebstemperaturen der Infrarotheizmodule derart zumindest teilweise unterschiedlich eingestellt oder einstellbar sind, dass die Intensität der von der Vorrichtung bereitgestellten Wärmestrahlung zum Zuführen an die Hautpartie zumindest entlang einer ersten und einer zweiten jeweils parallel zur spezifischen Transversalebene verlaufenden Richtung innerhalb zumindest eines betrachteten Bereichs außerhalb der Vorrichtung homogen ist.

15. Vorrichtung nach einem der vorangehenden Ansprüche, wobei sich die Infrarotheizmodule entlang einer zur spezifischen Transversalebene senkrecht verlaufenden Richtung streifenförmig erstrecken.

Fig. 1a

Fig. 1b

Fig. 2

Fig. 3

Fig. 4

Fig. 5

Fig. 6a

103'

109'

117'

115b'

107'

105'

113'

115a'

Fig. 6b

101'

103'

119'

119'

105'

107'

105'

109', 115b'

Fig. 7

123

Fig. 8

Fig. 9

Fig. 10a

Fig. 10b

Fig. 10c

Fig. 10d

Fig. 11

Fig. 12

Europäisches
Patentamt

European
Patent Office

Office européen
des brevets

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 107 096 133 A (CHONGQING YONGSHOUTANG FITNESS EQUIPMENT CO LTD) 29. August 2017 (2017-08-29) | 1-8, 10-13 | INV. A61N5/06 |
| Y | * Absätze [0016] - [0021] * * Abbildung 1 * ----- | 9 | ADD. A61F7/00 A47C7/74 |
| X | CN 107 097 701 A (UNIV ANHUI SCIENCE & TECH) 29. August 2017 (2017-08-29) * Absätze [0029] - [0041] * * Abbildungen * ----- | 1-8, 10-13 | |
| X | CN 202 666 205 U (WEIHAI SANMEI MEDICAL EQUIPMENT CO LTD) 16. Januar 2013 (2013-01-16) * Absätze [0011], [0012] * * Abbildungen * ----- | 1-8, 10-12 | |
| X | CN 113 230 082 A (GUANGDONG WENDAO BAIYI HEALTH TECH CO LTD) 10. August 2021 (2021-08-10) * Absätze [0045] - [0056] * * Abbildung 3 * ----- | 1-8, 10-13 | RECHERCHIERTE SACHGEBIETE (IPC) |
| X | CN 111 493 567 A (ZHU ZHIMING) 7. August 2020 (2020-08-07) * Absätze [0029] - [0036] * * Abbildungen * ----- | 1-8, 10-13 | A61N A61F A47D A47C |
| X | KR 2016 0066533 A (KIM HAN IL [KR]) 10. Juni 2016 (2016-06-10) * Absätze [0110] - [0135] * * Abbildung 8c * ----- | 1-8, 10-13 | |
| Y | RO 131 318 A2 (RENAULT TECH ROUMANIE S R L [RO]) 30. August 2016 (2016-08-30) * S. 5, sechster voller Absatz * ----- | 9 | |

-/--

Der vorliegende Recherchenbericht wurde für alle Patentansprüche erstellt

| Recherchenort | Abschlußdatum der Recherche | Prüfer |
|---|---|---|
| München | 10. Februar 2022 | Lohmann, Stefan |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

Seite 1 von 2

Europäisches
Patentamt

European
Patent Office

Office européen
des brevets

# EUROPÄISCHER RECHERCHENBERICHT

**Nummer der Anmeldung**

**EP 21 19 3188**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | DE 10 2007 027682 A1 (KELLER RAINER [DE]) 18. Dezember 2008 (2008-12-18) * das ganze Dokument * ----- | 1-15 | |

RECHERCHIERTE
SACHGEBIETE (IPC)

Der vorliegende Recherchenbericht wurde für alle Patentansprüche erstellt

| Recherchenort | Abschlußdatum der Recherche | Prüfer |
|---|---|---|
| München | 10. Februar 2022 | Lohmann, Stefan |

KATEGORIE DER GENANNTEN DOKUMENTE

X : von besonderer Bedeutung allein betrachtet
Y : von besonderer Bedeutung in Verbindung mit einer
      anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
      nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes
      Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 21 19 3188

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-02-2022

| Im Recherchenbericht angeführtes Patentdokument | Datum der Veröffentlichung | Mitglied(er) der Patentfamilie | Datum der Veröffentlichung |
|---|---|---|---|
| CN 107096133 A | 29-08-2017 | KEINE | |
| CN 107097701 A | 29-08-2017 | KEINE | |
| CN 202666205 U | 16-01-2013 | KEINE | |
| CN 113230082 A | 10-08-2021 | KEINE | |
| CN 111493567 A | 07-08-2020 | KEINE | |
| KR 20160066533 A | 10-06-2016 | KEINE | |
| RO 131318 A2 | 30-08-2016 | KEINE | |
| DE 102007027682 A1 | 18-12-2008 | KEINE | |

EPO FORM P0461

Für nähere Einzelheiten zu diesem Anhang : siehe Amtsblatt des Europäischen Patentamts, Nr.12/82